# EUROPEAN PATENT APPLICATION

(11) **EP 4 378 417 A1**
(43) Date of publication of application: **05.06.2024**
(21) Application number: 22849882.0
(22) Date of filing: 27.07.2022
(51) Int. Cl.: A61C 13/00, A61C 9/00, G06T 19/20, G06T 17/00, G16H 50/50, G16H 30/00, A61C 8/00, A61C 5/70, A61C 1/08

(54) **METHOD AND DEVICE FOR RECOMMENDING VIRTUAL ABUTMENT FOR DESIGNING SURGICAL GUIDE**

(30) Priority: 28.07.2021 KR 20210099476
(71) Applicant: Osstemimplant Co., Ltd., Gangseo-gu Seoul 07789 (KR)
(72) Inventor: KIM, Jongmoon, Gunpo-si Gyeonggi-do 15827 (KR); CHO, Sanghyeong, Seoul 07598 (KR); CHOI, Kyoo Ok, Seoul 07789 (KR)
(74) Representative: Weickmann & Weickmann PartmbB
(86) International application number: PCT/KR2022/011044
(87) International publication number: WO 2023/008906

(57) **Abstract**

A method for recommending a virtual abutment for designing a surgical guide according to one embodiment of the present invention may comprise the steps in which: a design device arranges a virtual crown in dental image data; a virtual implant body is designed according to the arrangement of the virtual crown; when the virtual abutment is set as a patient-customized abutment, at least one of the outer length of a gum part of the patient-customized abutment, the outer shape of the gum part, the diameter of the patient-customized abutment, or the crown insertion height (abutment height, A/H) value of the patient-customized abutment is determined on the basis of the virtual crown and virtual implant body; and an abutment that matches the patient-customized abutment is searched for in a pre-stored abutment library on the basis of the determination.

## Description

### Technical Field

The present disclosure relates to a method and device for recommending a virtual abutment for designing a surgical guide.

### Background Art

In general, designing a surgical guide for a dental implant using surgical guide software may allow a user to place an implant body at a correct position and achieve a high mechanical fixing force, and the user may thereby fix an abutment immediately after placing the implant body and fix a temporary prosthesis to a patient. Accordingly, dental implant surgery using surgical guide design software may provide patients with a sense of satisfaction in both aesthetical and functional aspects.

### Disclosure of Invention

### Technical Goals

An aspect of the present disclosure may provide an implant surgical guide design method and device that recommends a virtual abutment to a user when designing a dental implant surgical guide.

However, the problems to be solved by the present disclosure are not limited to those described above, and other problems to be solved that have not been described will be apparent to one of ordinary skill in the art from the following description.

### Technical Solutions

According to an embodiment of the present disclosure, there is provided a method of recommending a virtual abutment for designing a surgical guide, the method including: by a design device, placing a virtual crown on an image screen displaying dental image data; designing a virtual implant body according to the placement of the virtual crown; when an abutment included in an implant is set as a patient-specific abutment, determining at least one of an outer length of a gingival portion of the patient-specific abutment, an outer shape of the gingival portion, a diameter, or a crown insertion height (an abutment height A/H), based on the virtual crown and the virtual implant body; and searching a prestored abutment library for an abutment matching the patient-specific abutment based on the determining.

The searching may include: calculating a similarity between the patient-specific abutment and each abutment included in the abutment library, based on the determining; and determining the abutment matching the patient-specific abutment from the abutment library, based on the similarity.

The calculating of the similarity may include: calculating at least one of a first similarity between a gingival height G/H of the patient-specific abutment and a G/H of each abutment included in the abutment library, a second similarity between a diameter value of the patient-specific abutment and a diameter value of each abutment included in the abutment library, or a third similarity between the A/H of the patient-specific abutment and an A/H of each abutment included in the abutment library.

The determining of the abutment matching the patient-specific abutment may include: comparing the calculated at least one of the first similarity, the second similarity, or the third similarity to a preset reference value for the at least one, and determining the abutment matching the patient-specific abutment.

When an A/H of the abutment determined to match the patient-specific abutment is greater than the A/H of the patient-specific abutment, the method may further include: modifying the A/H of the abutment determined to match the patient-specific abutment, based on at least one of a strength of a temporary prosthesis or a material of a final prosthesis.

When the abutment included in the implant is set as a ready-made abutment, the method may further include: searching a prestored abutment library for an abutment matching the ready-made abutment, based on an angle between a vertical line from an uppermost end of the virtual implant body to the virtual crown and a line from the uppermost end of the virtual implant body to a margin portion of the virtual crown.

The method may further include: placing the virtual crown in the dental image data; designing the virtual implant body according to the placement of the virtual crown; when a placement position of the virtual implant body is moved, adjusting at least one of the outer length of the gingival portion of the patient-specific abutment, the outer shape of the gingival portion, the diameter, or the A/H, based on the moved placement position of the virtual implant body.

The method may further include: when the placement position of the virtual implant body is moved, changing a shape of the virtual crown based on the moved placement position of the virtual implant body.

According to another embodiment of the present disclosure, there is provided a method of generating a surgical guide performed by a design device, the method including: placing a virtual crown in dental image data; designing a virtual implant body according to the placement of the virtual crown; when a virtual abutment is set as a patient-specific abutment, determining at least one of an outer length of a gingival portion of the patient-specific abutment, an outer shape of the gingival portion, a diameter, or an abutment height A/H, based on the virtual crown and the virtual implant body; searching a prestored abutment library for an abutment matching the patient-specific abutment based on the determining; and applying the retrieved abutment to the surgical guide.

According to still another embodiment of the present disclosure, there is provided a method of recommending a virtual abutment for designing a surgical guide, the method including: by a design device, receiving an input as to whether the virtual abutment is a patient-specific abutment; placing a virtual crown in dental image data; designing a virtual implant body according to the placement of the virtual crown; verifying whether the inputted virtual abutment is set as the patient-specific abutment; and based on the placed virtual crown and the placed virtual implant body, searching a prestored abutment library for an abutment matching the patient-specific abutment when the virtual abutment is verified as the patient-specific abutment, and searching a prestored abutment library for an abutment matching a ready-made abutment when the virtual abutment is not verified as the patient-specific abutment.

According to yet another embodiment of the present disclosure, there is provided a device for recommending a virtual abutment for designing a surgical guide, the device including: a data acquisition unit configured to obtain dental image data; and a control unit configured to: when the virtual abutment is set as a patient-specific abutment, place a virtual crown using the image data; design a virtual implant body according to the placement of the virtual crown; determine at least one of an outer length of a gingival portion of the patient-specific abutment, an outer shape of the gingival portion, a diameter, or an abutment height A/H, based on the virtual crown and the virtual implant body; and search a prestored abutment library for an abutment matching the patient-specific abutment based on the determining.

### Effects of Invention

According to embodiments of the present disclosure, a user may adjust a placement position of a virtual implant body and show a changing shape of a virtual crown when designing a dental implant surgical guide and may thereby examine beforehand a shape of a final prosthesis. In addition, after designing the virtual crown and the virtual implant body, suggesting a suitable abutment and applying it to a manipulator may provide user convenience.

### Brief Description of Drawings

FIG. 1 is a diagram showing a configuration of a guide design device for dental implant surgery according to an embodiment of the present disclosure.
FIG. 2 is a flowchart showing a series of steps of determining a type of abutment and designing and recommending a determined abutment according to an embodiment of the present disclosure.
FIG. 3 is a flowchart showing a series of steps of designing and recommending a ready-made abutment in the case of a process of designing a ready-made abutment.
FIG. 4 is a flowchart showing a series of steps of designing and recommending a patient-specific abutment in the case of a process of designing a patient-specific abutment.
FIG. 5 is a screen showing an example of placing a virtual crown at an opposing tooth according to an embodiment of the present disclosure.
FIG. 6 is a screen showing a cross-sectional computed tomography (CT) view obtained after the placement of a virtual crown according to an embodiment of the present disclosure.
FIG. 7 is a screen showing a cross-sectional CT view of an implant body placed based on a central axis of a virtual crown according to an embodiment of the present disclosure.
FIG. 8 is a screen showing an example of determining a height from an uppermost end of a virtual implant body to a gingival line according to an embodiment of the present disclosure.
FIG. 9 is a screen showing an example of generating a connection line that connects both connection points on a virtual implant body according to an embodiment of the present disclosure.
FIG. 10 is a screen showing an example of measuring a distance between a virtual implant body and a gingival line according to an embodiment of the present disclosure.
FIG. 11 is a screen showing an example of determining a gingival height (G/H) of an abutment in the case of a margin type being a sub-gingival margin (or sub-margin herein) according to an embodiment of the present disclosure.
FIG. 12 shows a sub-margin according to an embodiment of the present disclosure.
FIG. 13 shows a supra-gingival margin (or supra-margin herein) according to an embodiment of the present disclosure.
FIG. 14 is a screen showing an example of outputting margin information according to an embodiment of the present disclosure.
FIG. 15 is a screen showing an example of determining an abutment height (A/H) according to an embodiment of the present disclosure.
FIG. 16 is a screen showing another example of determining an A/H according to another embodiment of the present disclosure.
FIG. 17 is a screen showing an example of determining a diameter of an abutment according to an embodiment of the present disclosure.
FIG. 18 shows a method of selecting an abutment based on an angle formed by a vertical line from both connection points on a virtual implant body to a crown and a line from both connection points on the virtual implant body to a margin portion of the crown, according to an embodiment of the present disclosure.
FIG. 19 is a screen providing a manipulator along with an abutment product and its specifications according to an embodiment of the present disclosure.
FIG. 20 is a screen showing an example of designing a virtual drilling hole according to an embodiment of the present disclosure.
FIG. 21 is a screen showing an example of connecting a connection point on a virtual implant body and a point of a bone margin according to an embodiment of the present disclosure.
FIG. 22 is a screen showing an example of adjusting the shape of a second connection line using a virtual manipulator according to an embodiment of the present disclosure.
FIG. 23 is a screen showing an example of three-dimensionally measuring a distance from a first gingival line to a second gingival line according to an embodiment of the present disclosure.
FIG. 24 is a screen showing an example of determining an outer length of a gingival portion of a patient-specific abutment in the case of a margin type being a sub-margin according to an embodiment of the present disclosure.
FIG. 25 is a screen showing an example of determining an outer length of a gingival portion of a patient-specific abutment in the case of a margin type being a supra-margin according to an embodiment of the present disclosure.
FIG. 26 is a screen showing an example of determining an outer length of a gingival portion of a patient-specific abutment in the case of a margin type being an equi-gingival margin according to an embodiment of the present disclosure.
FIG. 27 shows an equi-gingival margin according to an embodiment of the present disclosure.
FIG. 28 shows a type of outer shape of a gingival portion of a patient-specific abutment according to an embodiment of the present disclosure.
FIG. 29 is a video screen providing a virtual manipulator for adjusting an outer shape of a gingival portion of a patient-specific abutment according to an embodiment of the present disclosure.
FIG. 30 shows a change in the diameter based on a margin type of a patient-specific abutment according to an embodiment of the present disclosure.
FIG. 31 is a screen showing an example of determining an A/H in an anterior region according to an embodiment of the present disclosure.
FIG. 32 is a screen showing an example of determining an A/H in a posterior region according to an embodiment of the present disclosure.
FIG. 33 is a screen showing an example of determining a final A/H in consideration of the strength of a virtual crown and the material of a final prosthesis according to an embodiment of the present disclosure.
FIG. 34 shows a method of matching a patient-specific abutment designed in accordance with an embodiment of the present disclosure and a ready-made abutment.
FIG. 35 is a screen presenting the specifications of a patient-specific abutment and providing a user with an abutment selection screen through a virtual manipulator according to an embodiment of the present disclosure.
FIG. 36 is a screen presenting the specifications of a matched ready-made abutment and providing a user with an abutment selection screen through a virtual manipulator according to an embodiment of the present disclosure.
FIG. 37 is a screen displaying a change in the shape of a virtual crown in response to a change in the placement position of a virtual implant body according to an embodiment of the present disclosure.

### Best Mode for Carrying Out Invention

The advantages and features of the present disclosure, and methods of achieving them, will become apparent with reference to the embodiments described in detail with reference to the accompanying drawings. However, the present disclosure is not limited to the embodiments described herein and may be implemented in many different forms. The embodiments are provided merely to complete the present disclosure and give a complete understanding of the scope of the present disclosure to one of ordinary skill in the art to which the present disclosure pertains, and the present disclosure is defined by the scope of the claims.

In the description of embodiments of the present disclosure, a detailed description of well-known related structures or functions will be omitted when it is deemed that such description will cause an ambiguous interpretation of the present disclosure. In addition, the following terms are defined in light of the features or functions of the embodiments of the present disclosure, which may vary depending on the intent of the user or operator or the convention. Therefore, the definitions should be taken in the context of the entire specification.

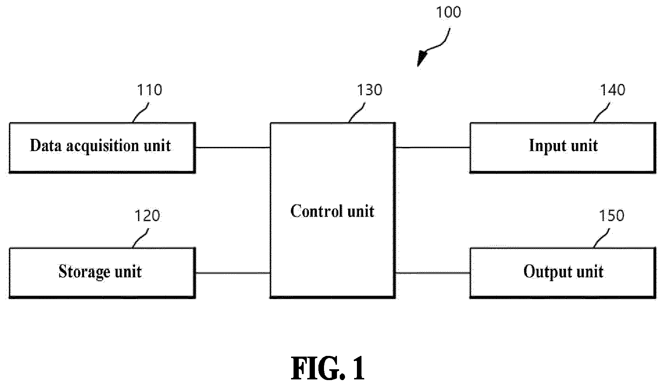

FIG. 1 is a diagram showing a configuration of a guide design device for dental implant surgery according to an embodiment of the present disclosure.

Referring to FIG. 1, a guide design device 100 may include a data acquisition unit 110, a storage unit 120, and a control unit 130. The guide design device 100 may further include an input unit 140 and an output unit 150.

The guide design device 100 may execute a variety of dental software for dental implant surgery. The dental software may include viewer software and computer-aided design (CAD) software. The viewer software and the CAD software may, according to circumstances, provide two-dimensional (2D) and three-dimensional (3D) images and may also provide diagnostic information about teeth and portions around the teeth.

The data acquisition unit 110 may obtain one or more pieces of data for dental implant surgery. The data may include, for example, computed tomography (CT) data, scan data. The CT data and the scan data may be used, individually or collectively, to design a surgical guide.

The scan data refers to data that includes information about actual teeth including a damaged tooth. The scan data may be obtained by scanning a plaster model of the patient's mouth using a 3D scanner or by scanning a dental impression of the patient's mouth using a 3D scanner. Alternatively, the scan data may be obtained by scanning the inside of the patient's mouth using a 3D intraoral scanner. The CT data refers to data generated in association with the patient's head portion using CT. The CT data may be obtained by a CT device, desirably, a cone beam CT (CBCT) device.

The storage unit 120 may have a plurality of libraries. The storage unit 120 may include at least one of, for example, a crown library, an abutment library, and an implant body library. These libraries may have information about crowns, abutments, or implant bodies associated respectively therewith. The crowns, abutments, and implant bodies stored in these libraries may then be processed by the control unit 130 or provided to the output unit 150.

In an embodiment of the present disclosure, the abutment library may be classified into a first abutment library and a second abutment library based on a margin type. Although the abutment library may be classified into at least three abutment libraries in consideration of diversification of the margin type, it may be classified into the first abutment library and the second abutment library in the embodiment based on the shape of a margin that is classified into a circular shape and a non-circular shape. That is, the first abutment library may be associated with abutments having a circular shape of a margin, and the second abutment library may be associated with abutments having a non-circular shape of a margin.

An abutment with a circular margin may be a ready-made abutment, and an abutment with a non-circular margin may be a patient-specific abutment. For reference, a margin of an abutment refers to a diametrically protruding portion, which may be classified as circular or non-circular according to its shape or may be classified as a supra-gingival margin (or simply "supra-margin" herein), equi-gingival margin, or sub-gingival margin (or simply "sub-margin" herein) according to its position in relation to the gingiva.

For the first abutment library, the specifications for abutments with a circular shape of a margin line may be predefined. For example, the first abutment library may be predefined by a gingival height (G/H), an abutment height (A/H), and a diameter. For the second abutment library, the specifications for abutments with a non-circular shape of a margin line may be predefined. For example, the second abutment library may be predefined by an outer length of the gingival portion, A/H, and diameter.

The control unit 130 may generate a virtual crown that matches an opposing tooth (or a matching tooth), in an area where the tooth is missing. The virtual crown may be generated to have the shape of a matching surface that is formed based on matching information about matching to the opposing tooth. In addition, the virtual crown may be generated to have the shape of a remaining portion, excluding the matching surface, that is formed using shape information about the same tooth or is selected from the crown library having a plurality of virtual crowns.

Based on the shape and position of the generated virtual crown, the control unit 130 may determine a virtual implant body corresponding to the virtual crown and determine a position of the virtual implant body. The control unit 130 may generate the virtual implant body having the determined shape at the determined position. The control unit 130 may generate, at the determined position, 3D data of the implant body itself or 2D data that represents two-dimensionally the implant body, according to circumstances.

The control unit 130 may then determine the specifications, or desirably measurements, of a virtual abutment based on at least two of the shape of the virtual implant body, the position of the virtual implant body, the shape of the virtual crown, and the position of the virtual crown, or desirably based on at least three thereof or more desirably based on at least four thereof. For reference, the specifications of a virtual abutment refer to all information associated with the shape of the abutment, and the measurements of a virtual abutment refer to measurable parameters in the specifications of the abutment. The specifications of an abutment may include, for example, a G/H, an A/H, a diameter, a perimeter of a margin, and a shape of a margin. Such abutment specifications may further include, for example, a shape of an upper margin portion that is above a margin with respect to a position of the margin, and a shape of a lower margin portion that is below the margin with respect to the position of the margin. As used herein, "above/up" refers to a direction from an abutment toward a crown, and "below/down" refers to a direction from an abutment toward an implant body.

The control unit 130 may search an abutment library for at least one abutment based on the abutment specifications and generate an execution screen including the retrieved abutment and/or its product code. In some cases, the retrieved abutment and/or its product code may be provided in a surgical report.

The control unit 130 may determine a change in a placement position of the virtual implant body. The change in a placement position of an implant body may include a change in a placement position of a virtual implant body, a change in a placement direction of the virtual implant body, and a change in a placement depth of the virtual implant body.

When the abutment is connected to the virtual implant body, the control unit 130 may calculate margin information, which is information about a boundary region between a virtual prosthesis and a tooth. In addition, the control unit 130 may check whether the placement position of the virtual implant body is changed and, when the placement position of the virtual implant body is changed, apply this change to recalculate the margin information.

The input unit 140 may be a means for receiving a user's operation signal and may include, for example, a touchscreen, a mouse, a keyboard, and a remote controller, but is not limited thereto. According to an embodiment, in a case in which the user desires to manually move the placement position of the virtual implant body, the input unit 140 may receive a user's operation signal for adjusting the placement position of the virtual implant body. The input unit 140 may also receive an input for selecting an abutment that the user desires to use from among recommended abutments output by the output unit 150.

The output unit 150 may display at least one of a simulation screen showing how the abutment is virtually placed, a simulation screen showing how the implant body and the abutment are engaged, or a simulation screen showing how the virtual crown is inserted into the top of the abutment. The output unit 150 may display, on a screen, optical abutment specifications determined through the control unit 130. In addition, it may display, on the screen, a color map representing in colors the margin information calculated through the control unit 130.

When the output unit 150 provides, on the screen, a virtual user interface for a user's operation and the input unit 140 receives a user's operation signal by an action performed by the user through the user interface, the control unit 130 may manually adjust the abutment. The virtual user interface may include a manipulator. The manipulator described herein may be provided to allow the user to adjust the abutment specifications according to an embodiment of the present disclosure. Further, the virtual manipulator may output recommended abutments and provide a screen from which the user may select an abutment.

Hereinafter, an abutment recommendation method implemented by a guide design device described above will be described. The abutment recommendation method may be implemented as at least a portion of a computer program. A control unit of the guide design device may use such software to recommend an abutment. An operation performed by the control unit may be described as an operation by the software.

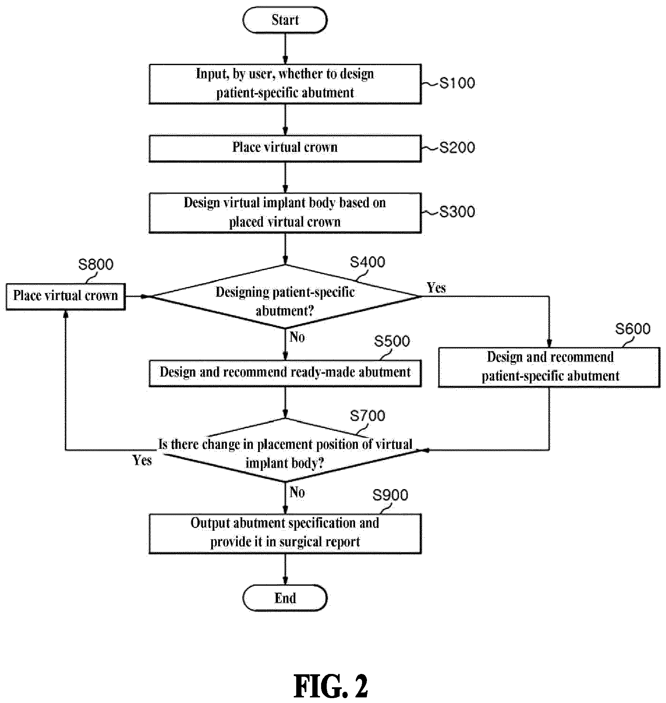

FIG. 2 is a flowchart showing a series of steps of determining an abutment specification according to an embodiment of the present disclosure.

Referring to FIG. 2, in step S 100, the software may receive an input on whether to design a patient-specific abutment. Based on the received input on whether to design the patient-specific abutment, a subsequent abutment design process will become different.

In step S200, the software may place a virtual crown at an opposing tooth.

In step S300, the software may determine a placement position, a placement direction, and a placement depth of a virtual implant body based on information associated with the placed virtual crown. In an embodiment, the information associated with the virtual crown may include specifications, tooth axis, and material information of the virtual crown. In some cases, the placement position, the placement direction, and the placement depth of the virtual implant body may change in response to user input.

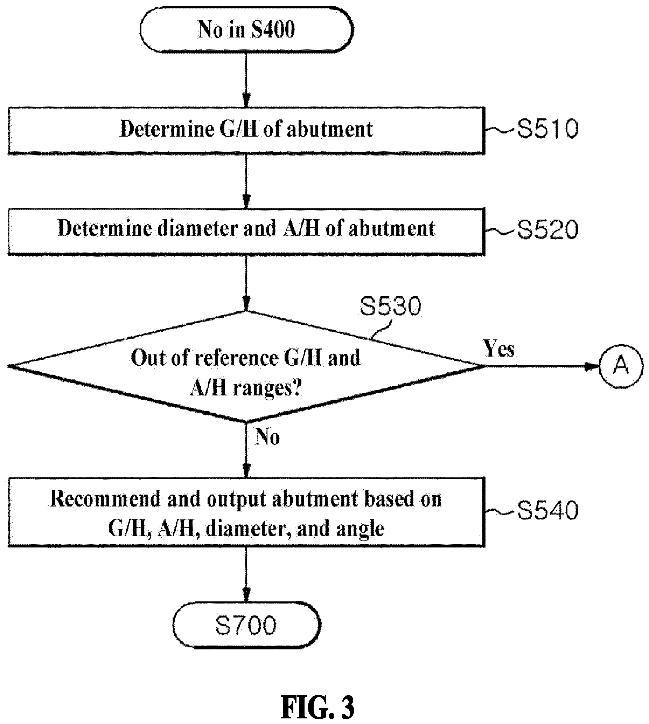

In step S400, the software may verify whether to design the patient-specific abutment. For example, the software may determine, as a design target, one from between the patient-specific abutment (or customized abutment) and a ready-made abutment based on user settings or user input. For another example, the software may calculate an abutment specification by preferentially selecting the ready-made abutment and, when the calculated specification is out of a reference range, determine to design the patient-specific abutment. In addition, in a case in which the ready-made abutment is preferentially selected, operation in step S540 may be immediately performed, without operations in steps S510 to S530 of S500 to be described below with reference to FIG. 3.

FIG. 3 is a flowchart showing a series of operations performed in step S500 of designing and recommending a ready-made abutment in a case of designing the ready-made abutment.

Referring further to FIG. 3, in step S510, when it is not a process of designing a patient-specific abutment ("No" in S400), the software may first determine a G/H of an abutment.

Subsequently, in step S520, when the G/H of the abutment is determined, the software may determine a diameter of the abutment by mesial, distal, buccal, and lingual widths of the crown, and may then determine, as an A/H of the abutment, a length from a margin line of the abutment to a highest part of the gingiva of the opposing tooth or a length to a lowest part of the virtual crown. In this case, the software may determine, as the A/H of the abutment, a height obtained by subtracting a preset value (e.g., 1.5 to 2.0 millimeters (mm)) in consideration of the strength or material of the crown.

Subsequently, in step S530, the software may check whether the determined G/H and A/H of the abutment are out of a preset reference range. When the determined G/H and A/H of the abutment are out of the preset reference range ("Yes" in S530), the software may not recommend an abutment product in the first abutment library, and the software may thus proceed to a process (e.g., S600) of designing a patient-specific abutment.

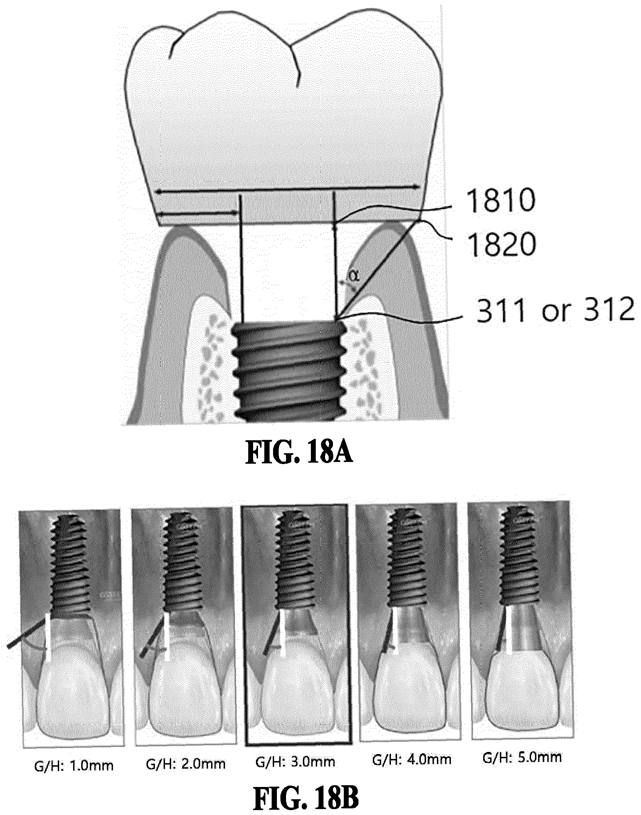

In step S540, when the determined G/H and A/H of the abutment are not out of the preset reference range ("No" in S530), the software may recommend a plurality of abutments in consideration of a G/H, a diameter, an angle, and a A/H of a designed abutment among ready-made abutments in the first abutment library. In this case, the angle of an abutment may be, for example, an angle α from a first connection point 311 or 312 of an uppermost end 310 of an implant body shown in FIG. 18 to the abutment.

For example, the software may first select a first group of abutments whose G/Hs satisfy a predetermined reference value from the first abutment library, and may then select a second group of abutments whose diameters are less than the diameter of the designed abutment from the selected first group. Subsequently, the software may select a third group of abutments whose angles are less than or equal to a predetermined value from the selected second group.

For example, in a case in which a margin type is a supra-margin, the software may select, as the first group, a plurality of abutments whose G/Hs are higher than the G/H of the designed abutment from the first abutment library. The software may then select, as the second group, abutments whose diameters are less than the diameter of the designed abutment from the selected first group.

Finally, the software may select the third group of abutments whose angles are less than or equal to the predetermined value from the selected second group. The number of a plurality of abutments in the third group to be recommended to the user by the software may be limited to three. In this case, a virtual manipulator may be displayed along with the plurality of recommended abutments. The software may select one from among the plurality of recommended abutments based on user input to the manipulator.

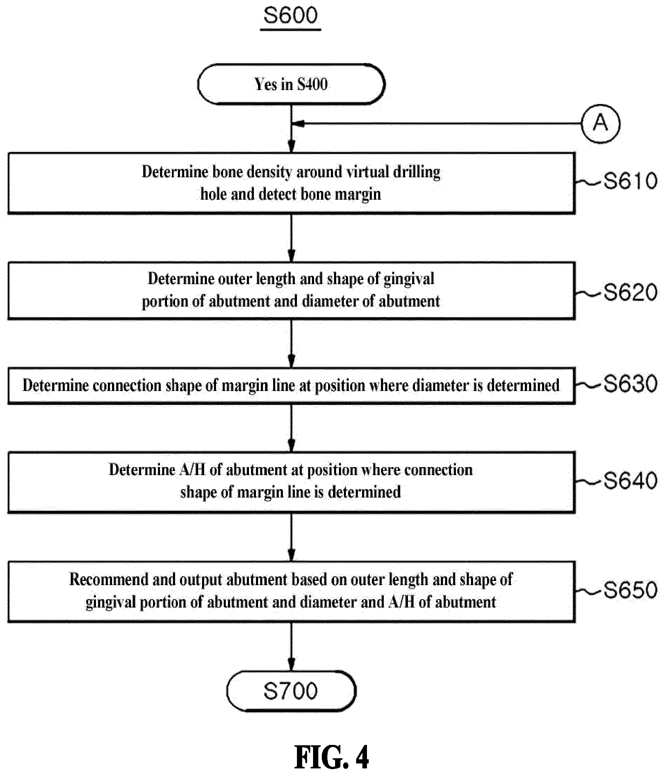

FIG. 4 is a flowchart showing a series of operations performed in step S600 of designing and recommending a patient-specific abutment in the case of designing the patient-specific abutment.

Referring further to FIG. 4, in step S610, in the case of designing a patient-specific abutment, the software may first determine a bone density around a virtual drilling hole and detect a bone margin.

Subsequently, in step S620, the software may determine an outer length and shape of a gingival portion of the abutment and a diameter of the abutment. When designing the shape of the gingival portion, the software may design the shape of a portion where the gingival portion of the abutment is adjacent to the bone margin and the shape of a portion where the gingival portion of the abutment is adjacent to a gingival margin.

Subsequently, in step 630, the software may determine a connection shape of a margin line of the abutment at a position where the diameter is determined. The connection shape of the margin line of the abutment refers to the shape of a portion of the margin line of the abutment that is connected to a prosthesis. In step S640, after determining an A/H of each of different abutments at the position where the connection shape of the margin line of the abutment is determined, the software may determine a final A/H of the abutment in consideration of the strength of the virtual crown and the material of a final prosthesis.

In step S640 of determining the A/H of the abutment, the software may determine the A/H of different abutments in consideration of at least one of the shape of the opposing tooth or the shape of the virtual crown, at a position where the connection shape of the margin line of the abutment is determined.

In addition, in step S640 of determining the A/H of the abutment, the software may determine, as the final A/H, a height obtained by subtracting a preset distance (e.g., 1.5 to 2.0 mm), in consideration of the strength of the virtual crown and the material of the final prosthesis.

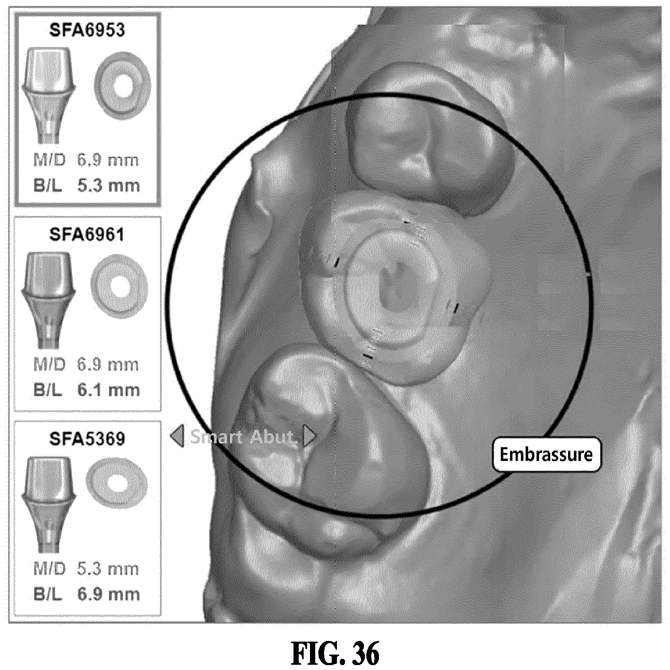

Subsequently, in step S650, the software may provide the user with the specifications of the designed patient-specific abutment without a change or recommend and output a plurality of ready-made abutments in the second abutment library. A method of selecting an abutment from the second abutment library will be described in detail below with reference to FIG. 36.

Referring back to FIG. 2, the software may check, on the output screen, a change in the placement position of the virtual implant body according to user input in step S700 and, when there is a change in the placement position of the virtual implant body, the software may place again the virtual crown in step S800 and redesign the abutment in step S500 or S600. That is, when there is a change in the placement position of the virtual implant body, the software may immediately recommend an abutment determined according to the change in the placement position of the virtual implant body to the user without executing a separate design program.

Subsequently, in step S900, when there is no change in the placement position of the implant body and the user selects an abutment, the software may output the specifications of the selected abutment and provide them in a surgical report. In this case, the software may record the specifications of the selected abutment in the surgical report and automatically link it to a product code.

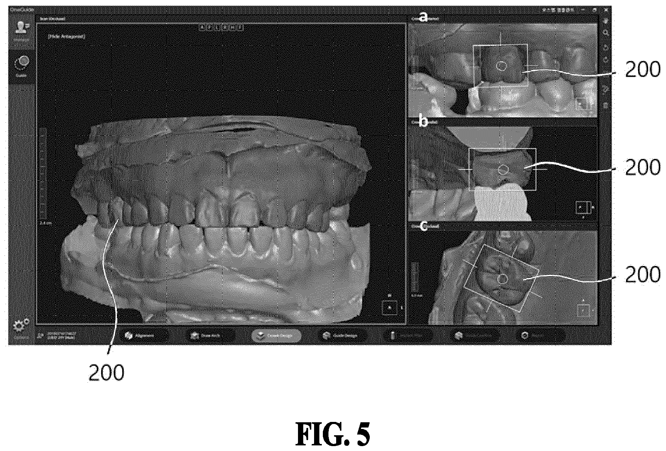

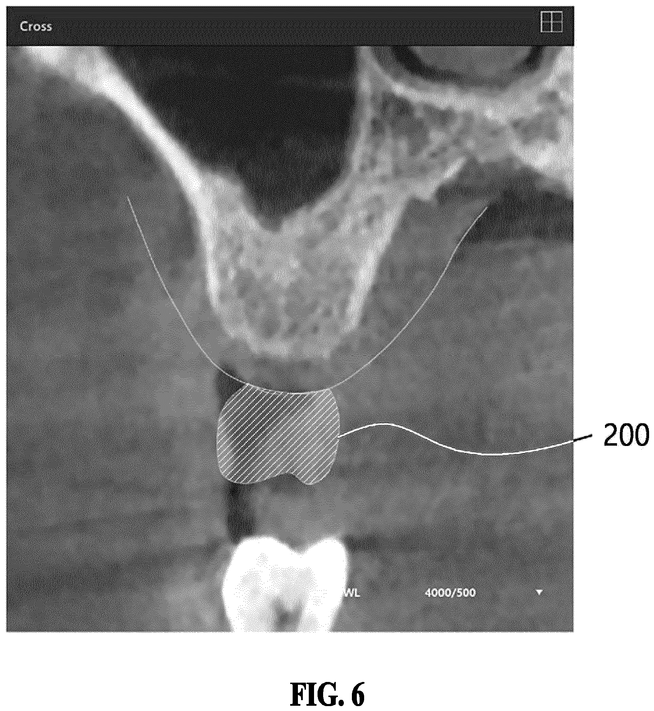

FIGS. 5 and 6 are screens showing a step of placing a virtual crown of FIG. 2.

FIG. 5 is a screen showing an example of placing a virtual crown according to an opposing tooth, according to an embodiment of the present disclosure.

Referring to FIG. 5, the software may place a virtual crown 200 on an opposing tooth. To this end, the software may match scan data and CT data of a patient to generate 3D oral data and may then place the virtual crown 200 on the opposing tooth using maxilla and mandible information in the 3D oral data.

For example, the mesial and distal widths of the virtual crown 200 may be set based on proximal teeth on both sides as shown in FIG. 5a, and the height of the virtual crown 200 may be set based on the shape of the opposing tooth from the gingiva in the scan data as shown in FIG. 5b. In addition, the buccal and lingual widths of the virtual crown 200 may be determined by the size of the buccal and lingual widths of the opposing tooth as shown in FIG. 5c.

FIG. 6 is a screen showing a cross-sectional CT view obtained after the placement of a virtual crown according to an embodiment of the present disclosure. Referring to FIG. 6, when a user places a virtual crown on 3D oral data by software or manually, the user may verify the placement of a virtual crown 200 on a CT screen (e.g., a cross-sectional view).

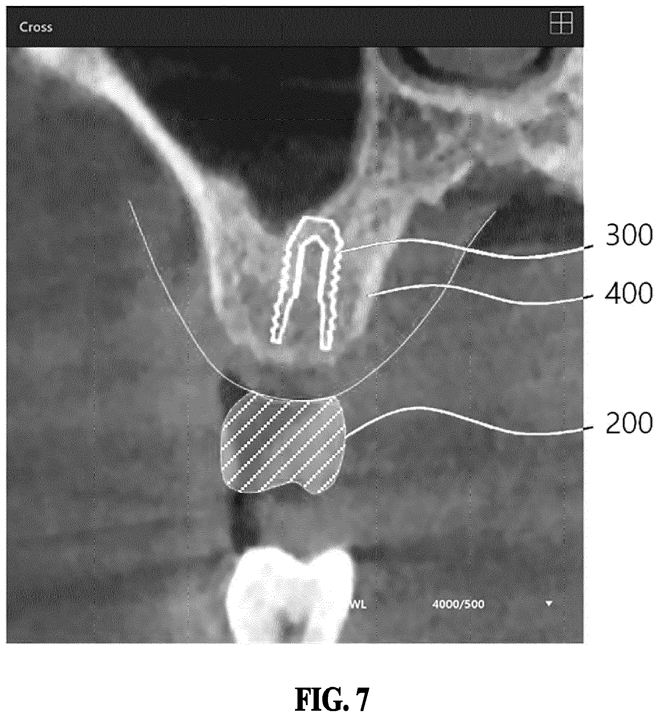

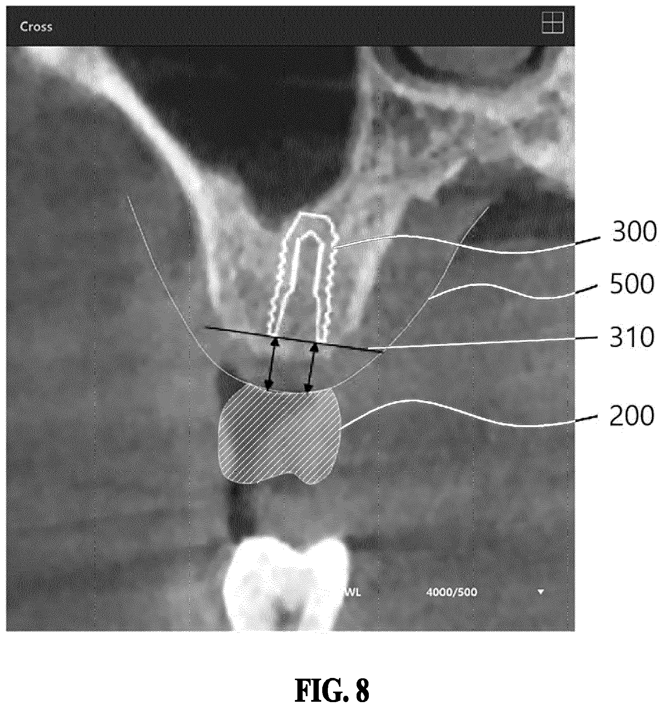

FIGS. 7 and 8 are video screens showing a step (e.g., S300 of FIG. 2) of designing a virtual implant body based on a placed virtual crown of FIG. 2 according to an embodiment of the present disclosure.

More specifically, FIG. 7 is a cross-sectional CT view of a virtual implant body placed with respect to a central axis of a virtual crown according to an embodiment of the present disclosure.

Referring to FIG. 7, the software may design a virtual implant body 300 including a placement position, a placement direction, and a placement depth of the virtual implant body 300, using a central axis of a virtual crown 200 and surrounding structures. For example, the software may determine the placement position of the virtual implant body 300 to be at the center of the virtual crown 200, with the virtual implant body 300 having the same central axis as the central axis of the virtual crown 200.

Further, the software may additionally consider information about a relationship between the virtual implant body 300 and the surrounding structures (e.g., alveolar bone, proximal teeth, and anatomical structures) to determine the placement position of the virtual implant body 300. For example, the software may determine the placement position of the virtual implant body 300 such that the virtual implant body 300 is positioned inside an alveolar bone 400. In this case, the software may determine the placement position of the virtual implant body 300 by checking alveolar bone information in the CT data.

Hereinafter, a step (e.g., S500 of FIG. 2) of designing and recommending a ready-made abutment will be described in detail with reference to FIGS. 8 through 19.

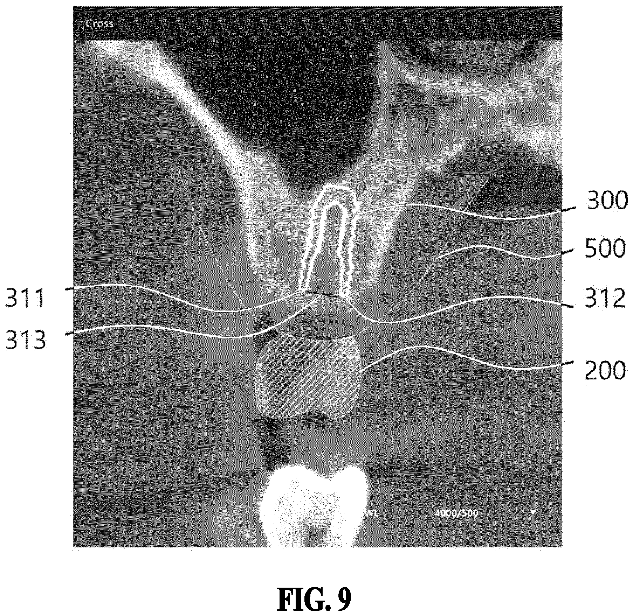

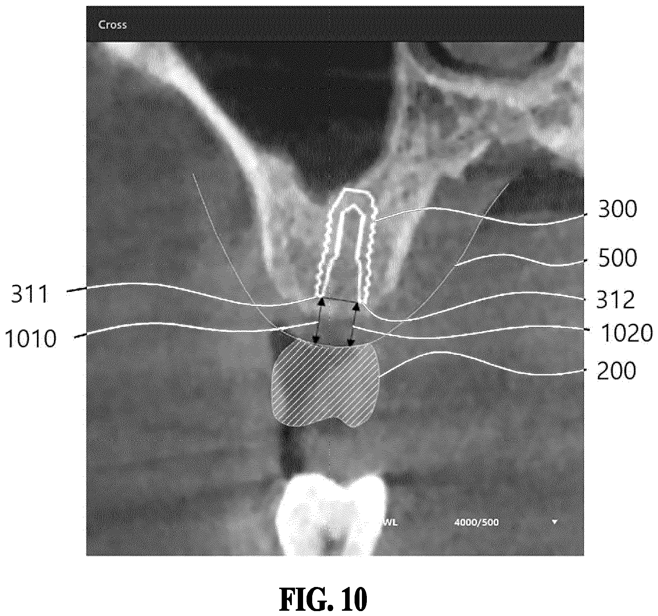

FIG. 8 is a screen showing an example of determining a height from an uppermost end of a virtual implant body 300 to a gingival line according to an embodiment of the present disclosure. FIG. 9 is a screen showing an example of generating a connection line that connects connection points on a virtual implant body 300 according to an embodiment of the present disclosure. FIG. 10 is a screen showing an example of measuring a distance between a virtual implant body 300 and a gingival line according to an embodiment of the present disclosure.

Referring to FIG. 8, when scan data and CT data are matched, margin information of the gingiva and cross-sectional information of the CT data in one of cross-sections of the CT data and oral model data are displayed together on the screen. In this case, the software may determine a height from an uppermost end 310 of the virtual implant body 300 displayed on the screen to a gingival line 500. As shown in FIG. 7, the virtual implant body 300 is positioned in the maxilla, and thus the top and bottom of an implant body are shown in an inverted form.

For example, as shown in FIG. 9, the software may generate a connection line 313 connecting first connection points 311 and 312 on the uppermost end 310 of the virtual implant body 300. The first connection points 311 and 312 may be where an abutment and the virtual implant body 300 contact, respectively.

As shown in FIG. 10, the software may measure vertical distances 1010 and 1020 between the first connection points 311 and 312 and the gingival line 500. The vertical distances 1010 and 1020 between the first connection points 311 and 312 and the gingival line 500 may be a height from the uppermost end 310 of the virtual implant body 300 to the gingival line 500.

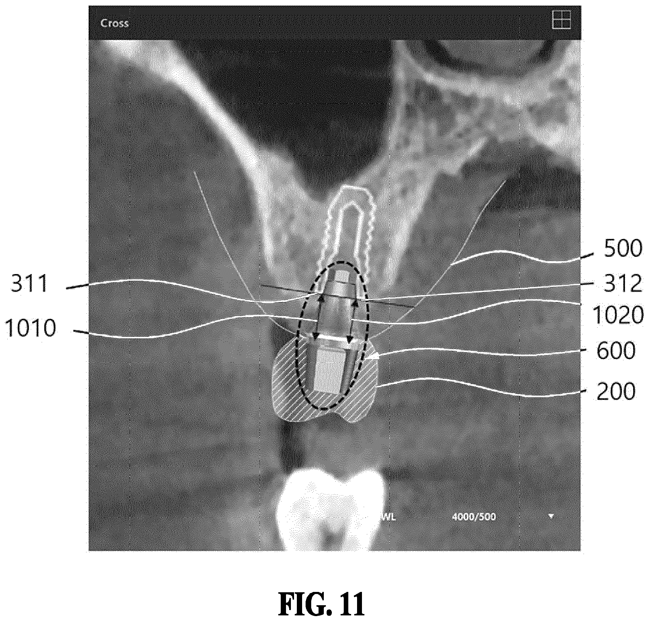

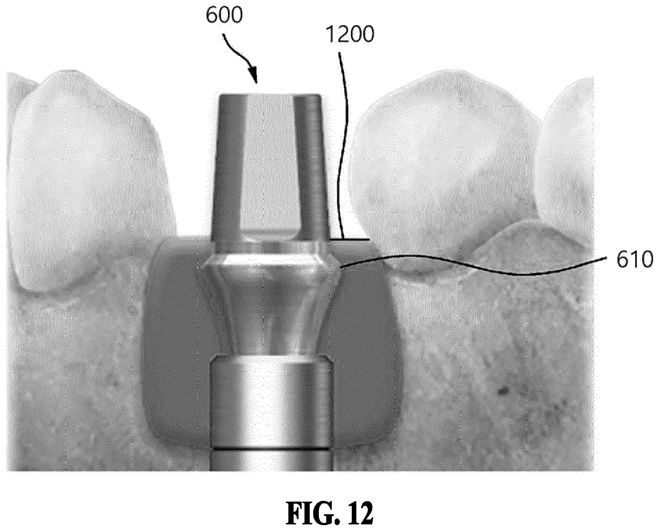

FIG. 11 is a screen showing an example of determining a G/H of an abutment in the case of a margin type being a sub-margin according to an embodiment of the present disclosure, and FIG. 12 shows a sub-margin according to an embodiment of the present disclosure.

Referring to FIGS. 11 and 12, in a case in which a margin type of an abutment 600 is a sub-margin, the software may determine a G/H of the abutment 600 such that a margin line 610 of the abutment 600 is to be positioned at a preset distance (e.g., 0.5 to 1.0 mm) from a gingival line 1200. The sub-margin may indicate that the margin line 610 of the abutment 600 is buried below the gingival line 1200 as shown in FIG. 12 and may generally be used in areas requiring aesthetics.

In this case, the software may select, from among a plurality of first abutments having a G/H that is less than the determined abutment G/H, an abutment having a G/H closest to the determined abutment G/H. The software may then determine the G/H of the selected abutment to be a G/H of an abutment being designed.

For example, as shown in FIG. 11, in a case in which the margin type is the sub-margin, the measured vertical distances 1010 and 1020 between the first connection points 311 and 312 and the gingival line 500 are 4.26 mm and 4.15 mm, respectively, and a G/H of a first abutment in the first abutment library is 3 mm, 4 mm, and 5 mm, the software may determine the G/H of the abutment to be 4 mm.

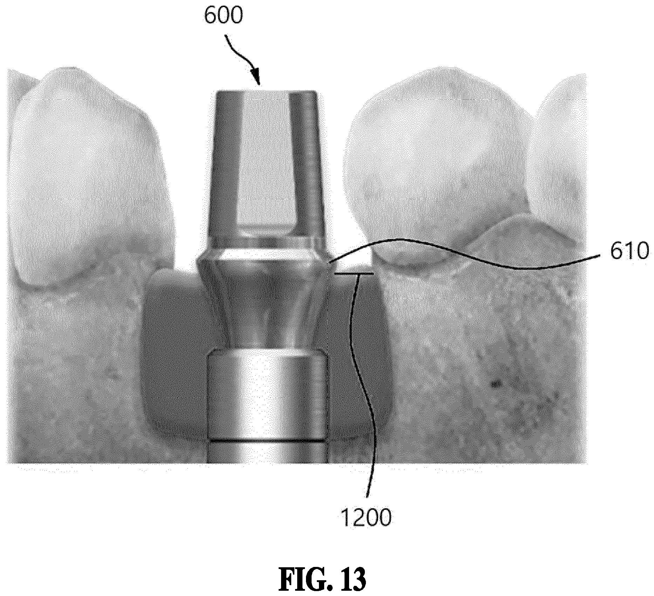

FIG. 13 shows a supra-margin according to an embodiment of the present disclosure.

Referring to FIGS. 11 and 13, in a case in which a margin type is a supra-margin, the software may determine a G/H of an abutment such that the margin line 610 of the abutment 600 is positioned at a preset distance (e.g., 0.5 to 1.0 mm) above the gingival line 1200, as shown in FIG. 13. The supra-margin may indicate that the margin line 610 of the abutment 600 is above the gingival line 1200 as shown in FIG. 13 and may generally be used in a molar teeth region to achieve desirable periodontal results.

In this case, the software may then select, from among a plurality of first abutments having a G/H that is greater than the determined abutment G/H, an abutment having a G/H closest to the determined abutment G/H. The software may then determine the G/H of the selected abutment to be a G/H of an abutment being designed.

For example, as shown in FIG. 13, in a case in which the margin type is the supra-margin, and the measured vertical distances 1010 and 1020 between the first connection points 311 and 312 and the gingival line 500 are 4.26 mm and 4.15 mm, respectively, and a G/H of a first abutment in the first abutment library is 3 mm, 4 mm, and 5 mm, the software may determine a G/H of an abutment to be 5 mm.

In a case in which the virtual implant body 300 needs to be deeply implanted or the gingiva is thick, and thus the G/H of the abutment 600 exceeds a preset maximum value (e.g., 7 mm), the software may generate warning information and output the generated warning information onto the screen. For example, the warning information may include a statement warning that there is a limit to selectable abutments.

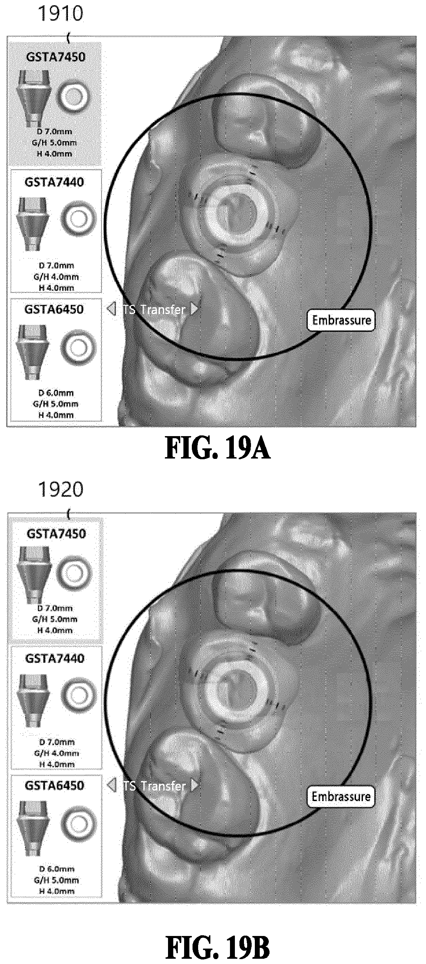

In this case, nothing may be displayed on a virtual manipulator for selecting an abutment specification, which will be described below with reference to FIG. 19, but an abutment recommended from the second abutment library, in lieu of the first abutment library, according to a process of designing a customized abutment, or a patient-specific abutment may be provided on a virtual manipulator.

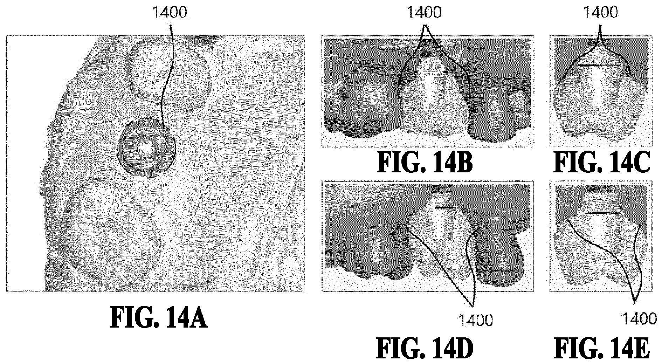

FIG. 14 is a screen showing an example of outputting margin information according to an embodiment of the present disclosure.

Referring to FIG. 14, in a case in which an abutment is connected to a virtual implant body according to a margin type set in advance by a user and displayed, margin information 1400 obtained according to a relationship between the virtual implant body and the abutment may be provided on the screen. The margin information 1400 may be provided in the form of a color map expressed in colors.

In addition, the software may provide a screen showing the margin information 1400 in various directions. For example, FIG. 14A shows a screen including margin information viewed from an occlusal direction, FIG. 14B shows a screen including margin information viewed from a buccal direction, FIG. 14C shows a screen including margin information viewed from a mesial direction, FIG. 14D shows a screen including margin information viewed from a lingual direction, and FIG. 14E shows a screen including margin information viewed from a distal direction.

After determining a G/H of an abutment, the software may determine a diameter of the abutment and then determine an A/H of the abutment. Alternatively, after determining the G/H of the abutment, the software may determine the A/H and then determine the diameter of the abutment. That is, the software may determine the G/H of the abutment first.

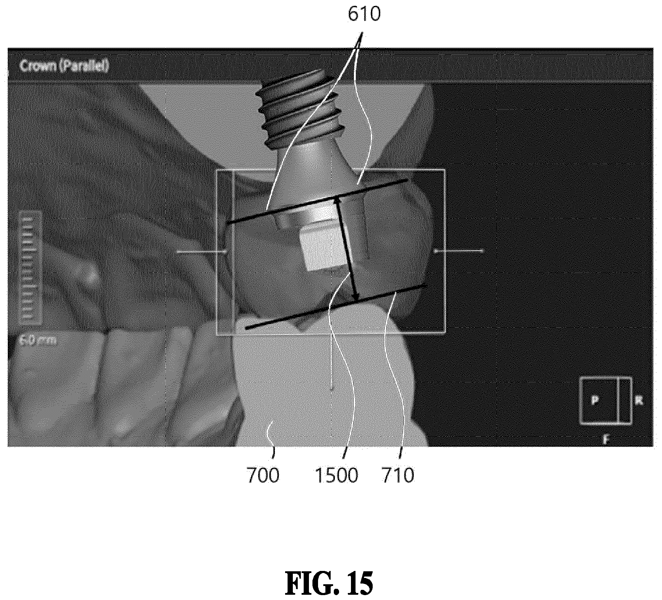

Hereinafter, an embodiment of determining an A/H of an abutment will be described with reference to FIGS. 15 to 17.

FIG. 15 is a screen showing an example of determining an A/H of an abutment according to an embodiment of the present disclosure.

Referring to FIG. 15, after determining a G/H of an abutment, the software may measure a vertical distance 1500 from a margin line 610 of the abutment to an uppermost end 710 of an opposing tooth 700 and determine an A/H of the abutment using the measured vertical distance 1500.

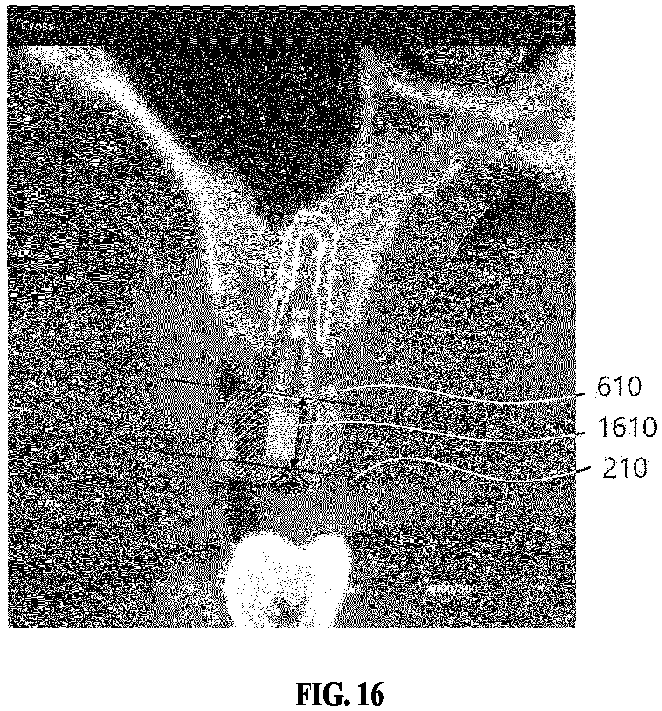

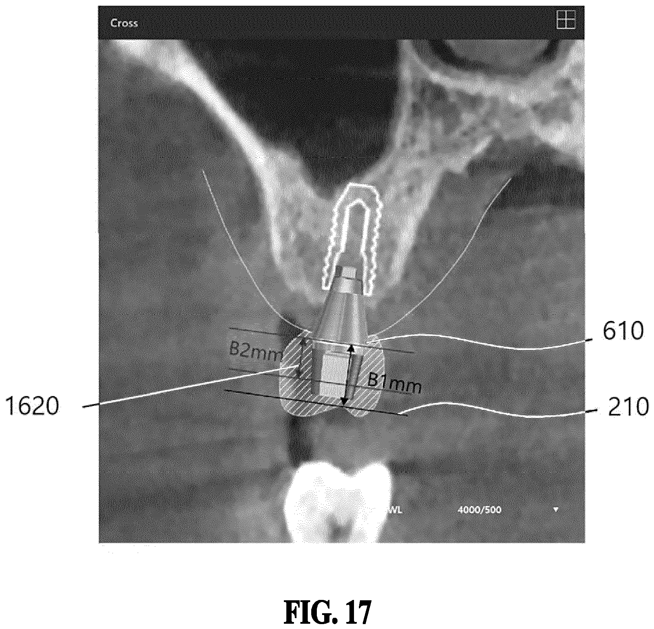

FIGS. 16 and 17 are screens showing another example of determining an A/H of an abutment according to another embodiment of the present disclosure.

Referring to FIGS. 16 and 17, after determining a G/H of an abutment, the software may measure a vertical distance 1610 from a margin line 610 of the abutment to an uppermost end 210 that is a position farthest from the gingiva of a virtual crown, and determine an A/H of the abutment using the measured vertical distance 1610 (e.g., B1). Subsequently, the software may determine, as the A/H of the abutment, a vertical distance 1620 (e.g., B2) obtained by subtracting a predetermined length in consideration of the strength of the virtual crown and the material of a final prosthesis. As shown in FIGS. 16 and 17, the virtual crown may be positioned in the maxilla, and thus the virtual crown may be provided in an inverted form.

After determining a G/H and an A/H of an abutment, the software may determine a diameter of the abutment using the mesial, distal, buccal, and lingual widths of a virtual crown. For example, while increasing the diameter of the abutment by a predetermined amount, the software may determine, as the diameter of the abutment, a length obtained at a point when the abutment contacts any one of the mesial, distal, buccal, and lingual widths of the virtual crown.

Alternatively, after determining a G/H and an A/H of an abutment, the software may measure the mesial, distal, buccal, and lingual widths of a crown. The software may then determine a diameter of the abutment based on the smallest one among the mesial, distal, buccal, and lingual widths of the crown. In a case in which, of a diameter of the virtual crown, the mesial, distal, buccal, and lingual widths do not all fall to an integer, the software may determine, as the diameter of the abutment, a value obtained by removing a decimal point from the measured (or determined) diameter value of the virtual crown.

FIG. 18 shows a method of selecting, from the first abutment library, one of which an angle (i.e., abutment angle) from the first connection points 311 and 312 of FIG. 10 to an abutment is less than or equal to a predetermined angle.

Referring to FIG. 18A, when an abutment in the first abutment library is combined with an implant body and a crown, the software may generate a first straight line perpendicularly connecting the first connection points 311 and 312 of the uppermost end 310 of the implant body to a point 1810 on the crown and a second straight line connecting the first connection points 311 and 312 of the uppermost end 310 of the implant body to a margin line 1820 of the abutment. Subsequently, the software may measure an angle α of the abutment, which indicates an angle between the first straight line and the second straight line, and may select only an abutment having the angle α that is less than or equal to a predetermined angle and provide the selected abutment to a user.

In this case, when the angle α of the abutment exceeds the predetermined angle, the gingiva may be pressed against the abutment, and thus pressure by the abutment may be applied to the gingiva. According to an embodiment, the predetermined angle may be 40 degrees (°).

For example, referring to FIG. 18B, the software may select an abutment having a G/H of 3.0 mm, 4.0 mm, and 5.0 mm from among abutments having a measured angle of 40° or less.

FIG. 19 is a screen providing a user with a virtual manipulator, as an output screen, including a determined abutment along with information such as product specifications according to an embodiment of the present disclosure.

Referring to FIG. 19, the software may provide a determined ready-made abutment to a user along with a product code or abutment specifications through a virtual manipulator and allow the user to select a product therefrom.

In this case, the determined ready-made abutment may further include, in addition to the most ideal ready-made abutment, the second most ideal ready-made abutment whose specifications are most similar to the most ideal ready-made abutment. In this case, the software may highlight (e.g., 1910) the most ideal ready-made abutment as shown in FIG. 19A to distinguish it from the second most ideal ready-made abutment, or may highlight (e.g., 1920) a color of an edge of a screen displaying the most ideal ready-made abutment as shown in FIG. 19B to distinguish it from the second most ideal ready-made abutment.

After determining the specifications (e.g., a G/H, an A/H, and a diameter) of an abutment, the software may record the specifications of the abutment in a surgical report in which even a product code is linked to the specifications of the abutment.

Depending on embodiments, the product code may be expressed in the following order: a predetermined management name (e.g., GSTA), the diameter, the A/H, and the G/H of the abutment. For example, in a case in which the specifications of the most ideal ready-made abutment selected by the user include an A/H of 4.0 mm, a diameter of 7.0 mm, and a G/H of 5.0 mm, the product code may be expressed as GSTA7450.

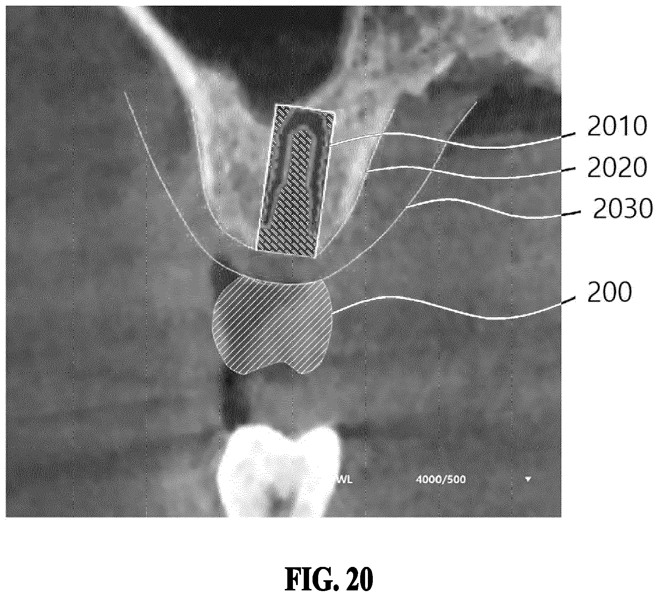

Hereinafter, a process (S600 of FIG. 2) of designing a patient-specific abutment will be described in detail with reference to FIGS. 20 to 36.

FIG. 20 is a screen showing an example of designing a virtual drilling hole according to an embodiment of the present disclosure.

Referring to FIG. 20, the software may analyze a bone density around a designed virtual implant body and design a virtual drilling hole 2010 based on a diameter and length of the virtual implant body.

The software may detect a bone margin and generate a first gingival line 2020 adjacent to the bone margin. The bone margin may be generated with reference to bone density information around the virtual drilling hole 2010 that is designed based on the virtual implant body.

For example, the bone density of a portion in contact with the virtual drilling hole 2010 may be obtained from CT data through a Hounsfield unit (HU) value of the portion in contact with the virtual drilling hole 2010, and the bone density of the gingiva may be obtained through an average HU value in the gingiva. A final bone margin may be obtained using the obtained bone density of the gingiva.

The software may also generate a second gingival line 2030 in contact with the virtual crown 200 from scan data.

In a customized abutment, or a patient-specific abutment herein, an outer length of a gingival portion of the abutment may include a first gingival portion outer length from a point where an implant body and the abutment are combined to a point where the first gingival line 2020 and the virtual drilling hole 2010 meet, and a second gingival portion outer length from a point where the first gingival line 2020 and the virtual drilling hole 2010 meet to a point where a virtual crown (e.g., the virtual crown 200 of FIG. 5) and the second gingival line 2030 meet. The first gingival portion outer length and the second gingival portion outer length may be designed respectively.

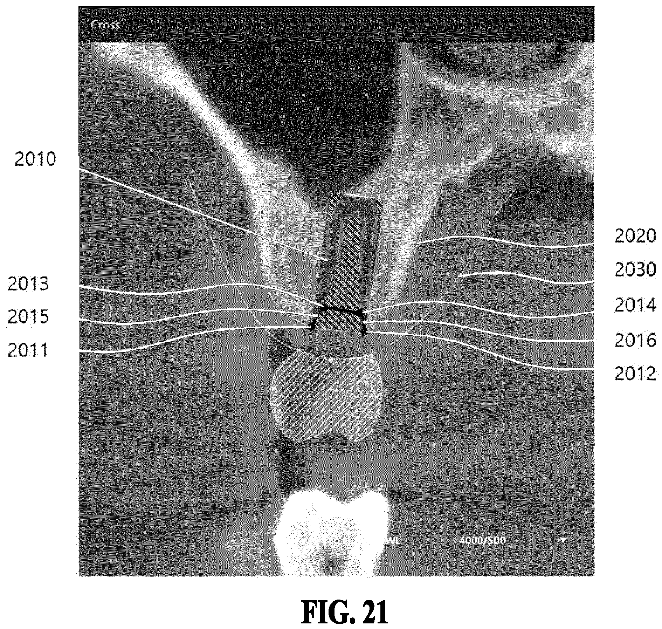

FIGS. 21 to 23 are screens showing a step (e.g., S620 of FIG. 4) of determining an outer length and shape of a gingival portion of an abutment and a diameter according to an embodiment of the present disclosure.

FIG. 21 is a screen showing an example of connecting a connection point of a virtual implant body and a point of a bone margin according to an embodiment of the present disclosure.

Referring to FIGS. 20 and 21, after matching scan data and CT data to generate 3D oral data, the software may provide a user with a screen that displays a first gingival line 2020 obtained from a cross section of the CT data and a second gingival line 2030 obtained from a cross section of the scan data.

The software may generate first connection points 2011 and 2012 at points of a virtual drilling hole 2010 where the virtual drilling hole 2010 and the first gingival line 2020 are in contact and may generate second connection points 2013 and 2014 at uppermost ends of an implant body, and may then generate a first connection line by connecting the second connection points 2013 and 2014.

Subsequently, the software may measure lengths from the first connection points 2011 and 2012 to the second connection points 2013 and 2014. In this case, the first connection points 2011 and 2012 may be positioned on the first gingival line 2020.

Also, the software may generate second connection lines 2015 and 2016 by connecting the second connection points 2013 and 2014 and the first connection points 2011 and 2012 of the virtual implant body. The second connection lines 2015 and 2016 may be straight or curved, and correspond to the first gingival portion outer length, which is a length from the uppermost end of the implant body to the first gingival line 2020.

In this case, each of a first connection point (e.g., 2011 and 2012) and a second connection point (e.g., 2013 and 2014) may be three or more. That is, although, because the screen shown in FIG. 21 is a 2D screen, the first connection point (e.g., 2011 and 2012) and the second connection point (e.g., 2013 and 2014) are each shown as two points for the convenience of description, each of the first connection point (e.g., 2011 and 2012) and the second connection point (e.g., 2013 and 2014) may each be provided as three or more points. Therefore, a second connection line (e.g., 2015 and 2015) connecting the first connection point (e.g., 2011 and 2012) and the second connection point (e.g., 2013 and 2014) may also be provided as three or more lines.

For example, a plurality of second connection lines (e.g., 2015 and 2016) may form a curved surface, and the curved surface may correspond to a surface including the first gingival portion outer length of the outer length of the gingival portion of the abutment.

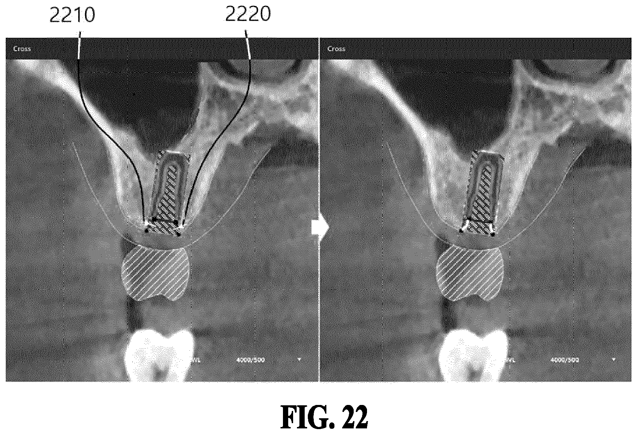

FIG. 22 is a screen showing an example of adjusting the shape of a second connection line using a virtual manipulator according to an embodiment of the present disclosure.

Referring to FIGS. 21 and 22, the software may provide a user with virtual manipulators 2210 and 2210 for manual adjustment at predetermined points (e.g., a 1/2 point, a 1/3 point, etc.) on the second connection lines 2015 and 2016 and may thereby allow the user to adjust the shape of the second connection lines 2015 and 2016.

According to an embodiment, as shown in FIG. 22, the software may provide a virtual manipulator that allows the user to adjust the shape of the second connection lines 2015 and 2016 to only a concave shape. That is, the user may adjust the shape of the second connection lines 2015 and 2016 from the concave shape to a straight shape.

In a case in which the user adjusts the second connection lines 2015 and 2016 to a convex shape, an abutment may be fastened to a bone around a drilling hole when an operator drills using a designed guide places an implant body, and fastens the abutment, and thus there may be a misconnection or there may be an inconvenience of having to remove the surrounding bone.

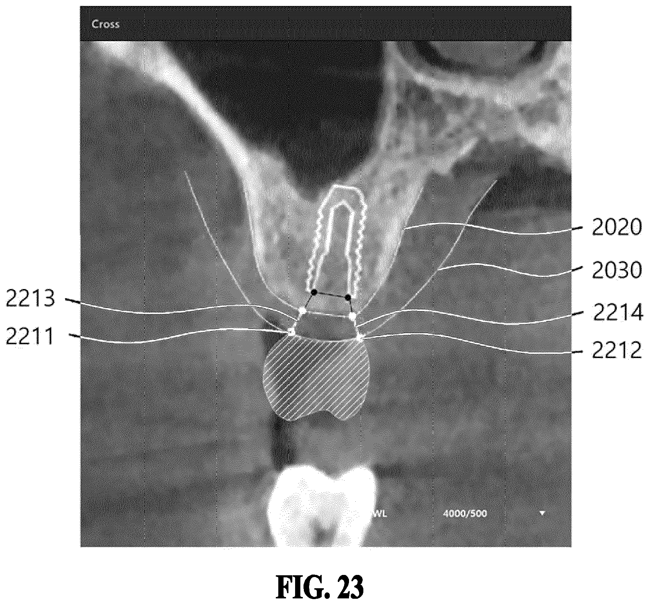

FIG. 23 is a screen showing an example of three-dimensionally measuring a distance from a first gingival line to a second gingival line according to an embodiment of the present disclosure.

Referring to FIGS. 21 to 23, the software may measure second gingival portion outer lengths 2213 and 2214 from the first connection points 2011 and 2012 on the first gingival line 2020 to third connection points 2211 and 2212 on the second gingival line 2030.

Depending on embodiments, the second gingival portion outer lengths 2213 and 2214 may be different from each other. For example, the second gingival portion outer lengths 2213 and 2214 may be determined to be 2.51 mm and 2.48 mm, respectively.

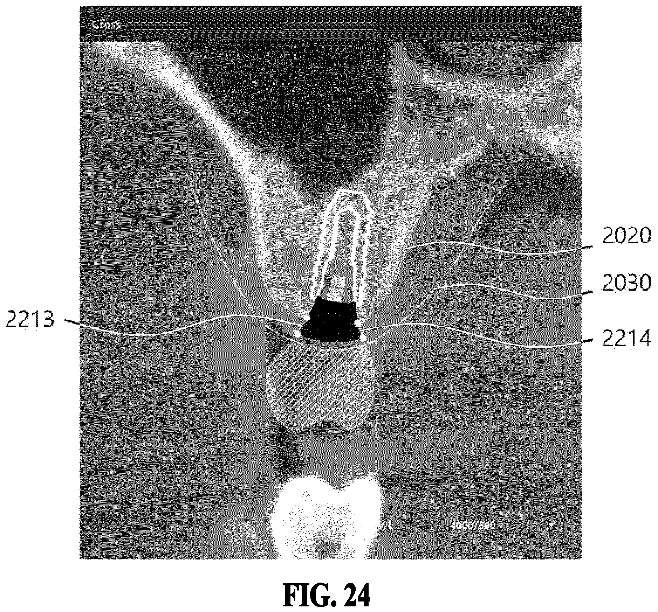

FIGS. 24 to 30 are screens showing a step (S620 of FIG. 4) of determining an outer length and shape of a gingival portion of an abutment, and a diameter of the abutment according to a margin type, according to an embodiment of the present disclosure.

When designing a patient-specific abutment, the software may adjust an outer shape of a gingival portion of the abutment, unlike when using a first abutment.

In general, a cross-sectional shape of the gingiva is U-shaped rather than a lineal shape, and it may thus be difficult to set an equi-gingival margin for the first abutment. In contrast, in a process of designing a patient-specific abutment, adjusting an outer shape of a gingival portion of the abutment may enable the unrestricted setting of a sub-margin, an equi-gingival margin, or a supra-margin.

For example, in a case in which a cross-sectional shape of the gingiva is U and the gingiva is positioned 0.5 mm below a setting line, the software may design a patient-specific abutment such that a margin line of the abutment is three-dimensionally U-shaped and is positioned 0.5mm below the setting line, thereby implementing the sub-margin.

FIG. 24 is a screen showing an example of determining an outer length of a gingival portion of a patient-specific abutment in a case in which a margin type is a sub-margin according to an embodiment of the present disclosure.

Referring to FIGS. 24 and 12, in a case in which a margin type is a sub-margin, the software may change the second gingival portion outer lengths 2213 and 2214 to be less than a value (e.g., 0.5 mm for the sub-margin) determined in a previous step, and for example, may change the second gingival portion outer lengths 2213 and 2214 from 2.51 mm to 2.01 mm and from 2.48 mm to 1.98 mm, respectively. In the case of the sub-margin, the abutment margin line 610 is below the gingival line 1200 and may thus be generally used in areas where aesthetics is considered important.

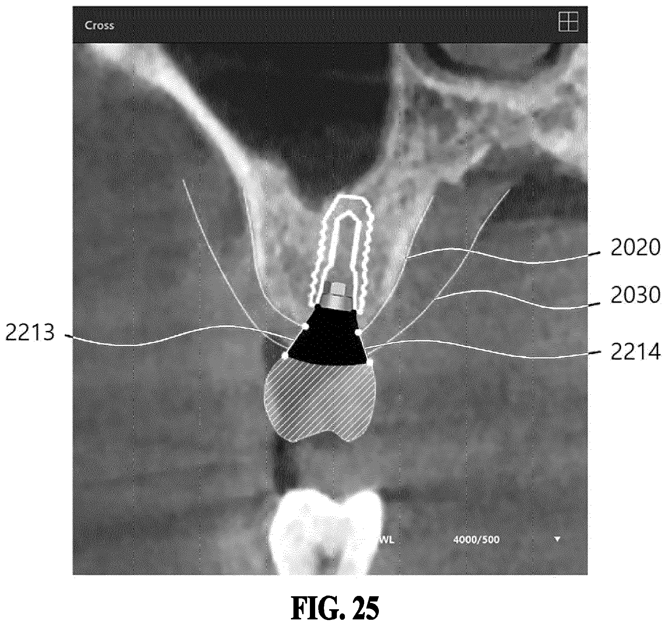

FIG. 25 is a screen showing an example of determining an outer length of a gingival portion of a patient-specific abutment in a case in which a margin type is a supra-margin according to an embodiment of the present disclosure.

Referring to FIGS. 25 and 13, in a case in which a margin type is a supra-margin, the software may change the second gingival portion outer lengths 2213 and 2214 to be greater than a value (e.g., 0.5 mm for the supra-margin) determined in a previous step, and for example, may change the second gingival portion outer lengths 2213 and 2214 from 2.51 mm to 3.01 mm and from 2.48 mm to 2.98 mm, respectively. In the case of the supra-margin, the abutment margin line 610 is above the gingival line 1200 and may thus be generally used in a molar teeth region and may correspond to a desirable periodontal margin.

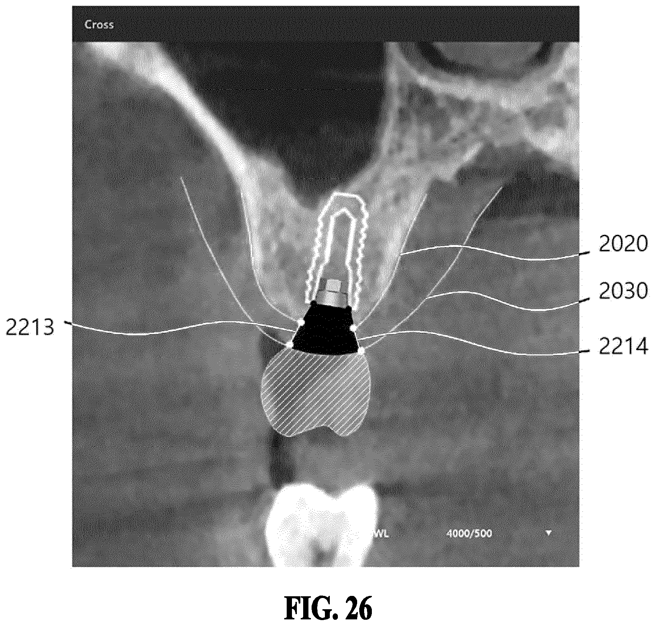

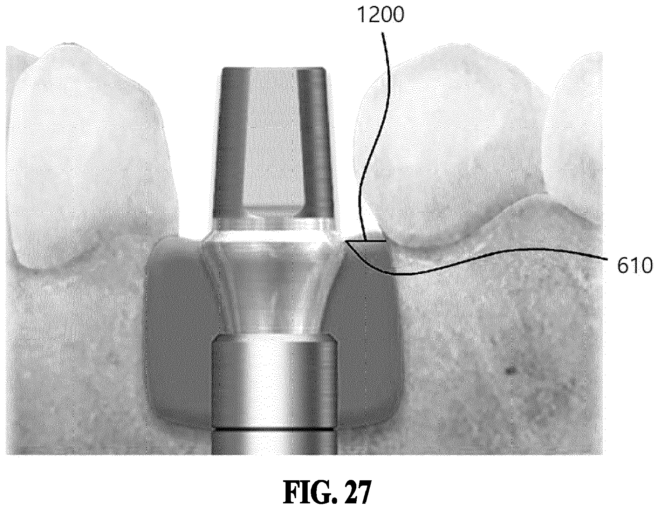

FIG. 26 is a screen showing an example of determining an outer length of a gingival portion of a patient-specific abutment in a case in which a margin type is an equi-gingival margin according to an embodiment of the present disclosure. FIG. 27 shows an equi-gingival margin according to an embodiment of the present disclosure.

Referring to FIGS. 26 and 27, in a case in which a margin type is an equi-gingival margin, a position of the gingival line 1200 and a position of the abutment margin line 610 may be matched, and thus the software may determine the second gingival portion outer lengths 2213 and 2214 to be the same as a value determined in a previous step.

In the case of the first abutment, the shape of the abutment margin line 610 is straight when viewed from the side, and it may thus be difficult to implement the equi-gingival margin. Therefore, the equi-gingival margin and a scalloped margin that implements the shape of the gingiva as it is may be applied only to a patient-specific abutment.

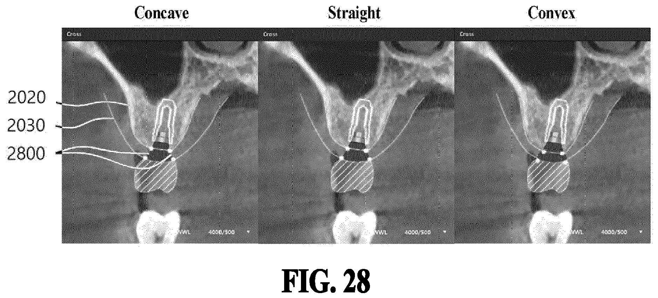

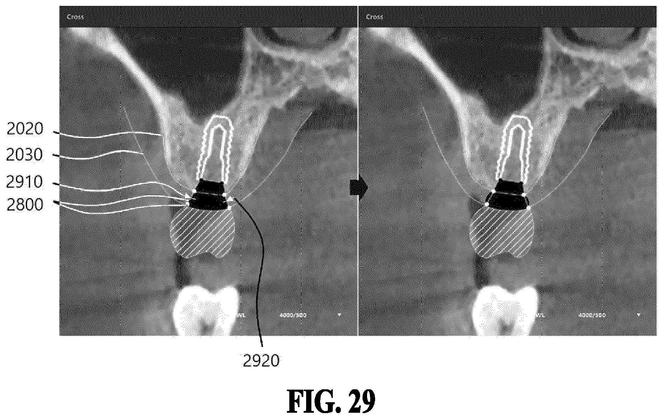

FIG. 28 shows a type of outer shape of a gingival portion of a patient-specific abutment according to an embodiment of the present disclosure, and FIG. 29 is a screen providing a virtual manipulator for adjusting an outer shape of a gingival portion of a patient-specific abutment according to an embodiment of the present disclosure.

Referring to FIGS. 26, 28, and 29, when designing a patient-specific abutment, the software may adjust an outer shape 2800 of the second gingival portion outer lengths 2213 and 2214 to be concave, straight, or convex.

To allow a user to adjust the outer shape 2800 of the gingival portion, the software may display virtual manipulators 2910 and 2920 for adjusting an outer shape of a gingival portion, at predetermined points on the second gingival portion outer lengths 2213 and 2214 (e.g., midpoints on the second gingival portion lengths 2213 and 2214, respectively). The user may then adjust the outer shape of the gingival portion of the abutment by a predetermined motion (e.g., expanding or reducing using a mouse drag method).

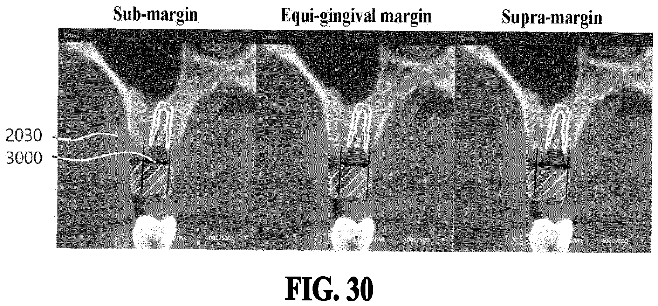

FIG. 30 shows a change in a diameter of a patient-specific abutment according to an embodiment of the present disclosure.

Referring to FIG. 30, unlike in a process of designing a ready-made abutment in the first abutment library having a fixed diameter value, the software may change a diameter of an abutment in a process of designing a patient-specific abutment.

Therefore, when designing a patient-specific abutment, the software may determine a diameter 3000 of the abutment based on a virtual crown generated in a virtual crown placement step (S200 of FIG. 2) and a margin type (i.e., supra-margin, sub-margin, and equi-gingival margin).

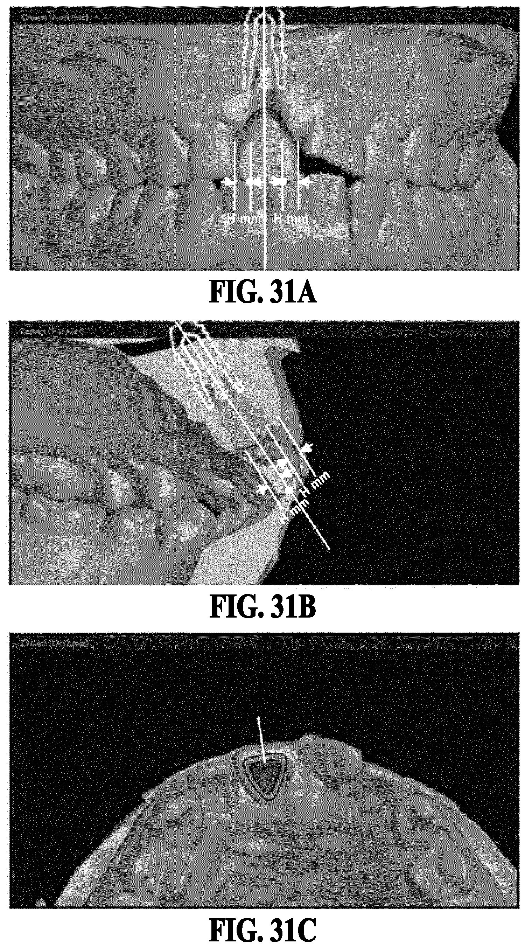

FIGS. 31 to 33 are screens showing a step (S640 of FIG. 4) of determining an A/H of an abutment.

FIG. 31 is a screen showing an example of determining an A/H of an abutment in an anterior region according to an embodiment of the present disclosure.

Referring to FIG. 31, in the anterior region, the software may measure an A/H of an abutment at a point moved by a preset length H mm (e.g., 1.5 to 2 mm) in a central axis direction (i.e., the mesial, distal, buccal, and lingual directions from four outer surfaces of a virtual crown, respectively) of the abutment from an outer surface of the designed virtual crown.

For example, according to an embodiment of the present disclosure, FIG. 31A shows an anterior view from which a point at which an A/H value of an abutment is to be measured is determined, FIG. 31B shows a cross-sectional view from which a point at which an A/H value of an abutment is to be measured is determined, and FIG. 31C shows an occlusal view from which a point at which an A/H value of an abutment is to be measured is determined.

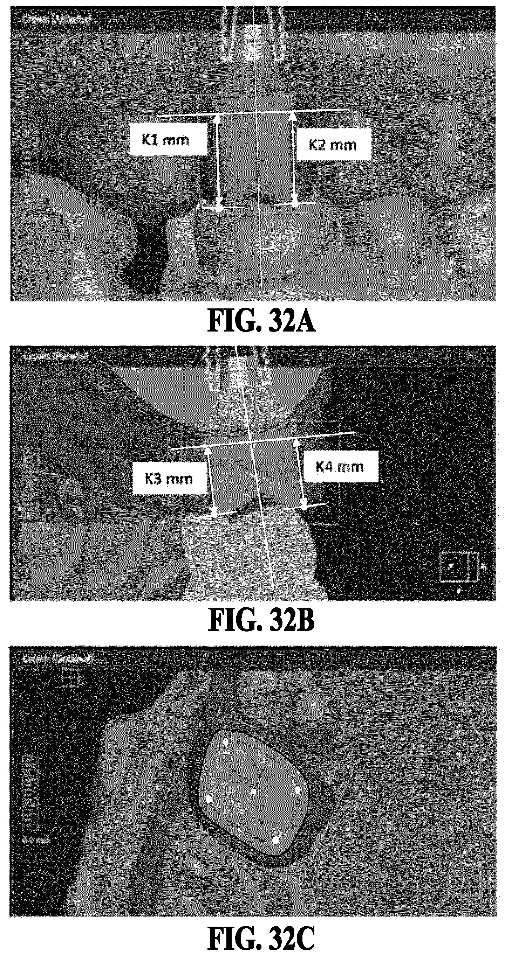

FIG. 32 is a screen showing an example of determining an A/H of an abutment in a posterior region according to an embodiment of the present disclosure.

Referring to FIG. 32, in the posterior region, the software may measure an A/H of an abutment based on the mesial, distal, buccal, and lingual cusps of a designed virtual crown. For example, according to an embodiment of the present disclosure, FIG. 32A shows an anterior view from which a point at which an A/H value of an abutment is to be measured is determined, FIG. 32B shows a cross-sectional view from which a point at which an A/H value of an abutment is to be measured is determined, and FIG. 32C shows an occlusal view from which a point at which an A/H value of an abutment is to be measured is determined.

In a process of designing a ready-made abutment, each value is determined in advance, and thus a value up to the highest part of an opposing tooth may be measured, or a value up to the lowest part of a virtual crown may be measured. In contrast, in the process of designing a patient-specific abutment, each value may be adjustable according to the shape of the opposing tooth or the shape of the virtual crown.

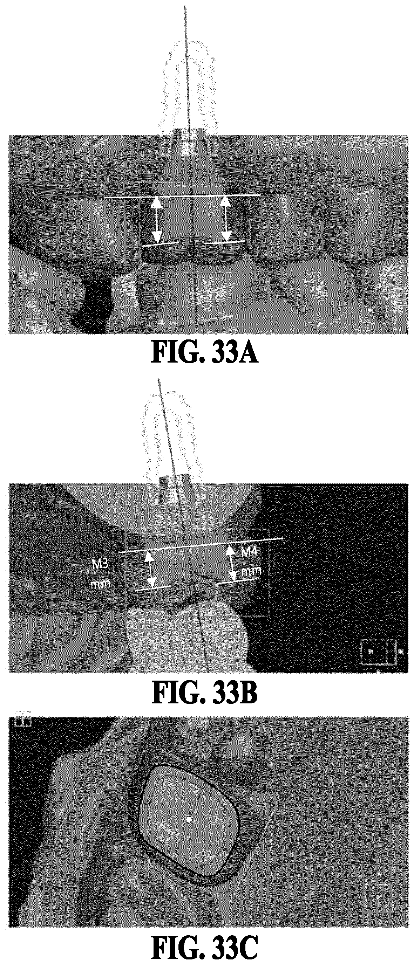

FIG. 33 is a screen showing an example of determining a final A/H of an abutment in consideration of the material or strength of a virtual crown according to an embodiment of the present disclosure.

Referring to FIG. 33, the software may determine a final A/H of an abutment by subtracting a preset length (e.g., 1.5 to 2.0 mm) from the measured A/H of the abutment in an abutment combined portion, in consideration of the strength of a temporary prosthesis (e.g., a temporary virtual crown) or the material of a final prosthesis. For example, the software may determine the final A/H (M1 mm to M4 mm) of the patient-specific abutment of FIG. 33 by reducing 2.0 mm from the A/H (K1 mm to K4 mm) of the abutment of FIG. 32.

For example, according to an embodiment of the present disclosure, FIG. 33A shows an anterior view from which a final A/H of an abutment is determined, FIG. 33B shows a cross-sectional view from which a final A/H of an abutment is determined, and FIG. 33C shows an occlusal view from which a final A/H of an abutment is determined.

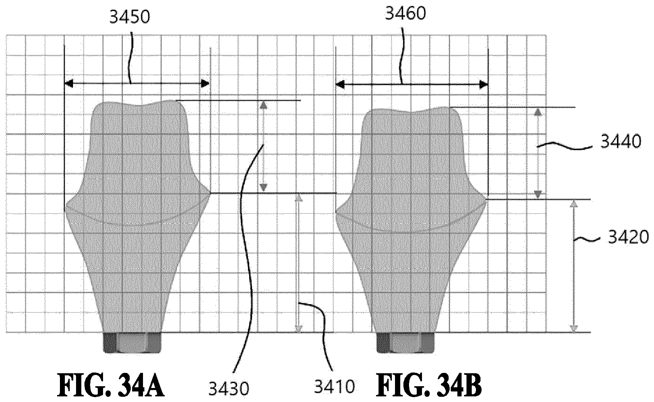

FIG. 34 shows a method of matching a specification of a designed patient-specific abutment to an abutment in the second abutment library according to an embodiment of the present disclosure.

Referring to FIGS. 4 and 34, step S650 of recommending and outputting an abutment in consideration of at least one of an outer length of a gingival portion of the abutment, an outer shape of the gingival portion, a diameter, or an A/H may include a step of matching a designed patient-specific abutment and an abutment in the second abutment library.

In the step of matching the designed patient-specific abutment and the abutment in the second abutment library, the software may first arrange, in a 3D coordinate system, the designed patient-specific abutment and the abutment in the second abutment library. The software may then compare a similarity between the designed patient-specific abutment and the abutment in the second abutment library that are arranged in the 3D coordinate system.

The abutment specifications for comparing the similarity may include G/Hs 3410 and 3420, A/Hs 3430 and 3440, and diameters 3450 and 3460, which are included in an outer shape of an abutment.

The software may compare the similarities, and match, to the designed patient-specific abutment, an abutment having a similarity that is greater than or equal to a predetermined value among abutments in the second abutment library.

More specifically, according to an embodiment of the present disclosure, the software may compare areas corresponding to the G/Hs 3410 and 3420 of the designed patient-specific abutment and the abutment in the second abutment library, and define a degree of similarity therebetween as a first similarity. The software may match, to the designed patient-specific abutment, an abutment in the second abutment library whose first similarity is greater than or equal to a predetermined value (e.g., 90%).

In addition, the software may compare the diameters 3450 and 3460 of the abutment to define a second similarity, and may match, to the designed patient-specific abutment, an abutment in the second abutment library whose second similarity is greater than or equal to a predetermined value (e.g., 90%).

Subsequently, the software may compare the A/Hs 3430 and 3440 of the abutment to define a third similarity, and may match, to the designed patient-specific abutment, an abutment in the second abutment library whose third similarity is greater than or equal to a predetermined value (e.g., 90%).

In this case, the software may define, as a first group, abutments having the first similarity that is greater than or equal to the predetermined value (e.g., 90%) among the abutments in the second abutment library and may define, as a second group, abutments having the second similarity that is greater than or equal to the predetermined value (e.g., 90%) among the abutments in the first group. Subsequently, the software may select, as a patient-specific abutment, an abutment having the third similarity that is greater than or equal to the predetermined value (e.g., 90%) among the abutments in the second group.

It may be relatively easy to change the length for the A/Hs 3430 and 3440, but it may not be easy to change the G/Hs 3410 and 3420 and the diameters 3450 and 3460 and they may be of importance. Therefore, the software or user may determine a matching order in consideration of this.

The software may select three matched abutments from the second abutment library, but embodiments are not limited thereto. That is, the number of abutments selected by the software may vary depending on embodiments.

In this case, when the A/Hs 3430 and 3440 of the selected abutment are greater than those of the designed patient-specific abutment, the software may provide the user with a modified A/H value (e.g., a reduced value) based on the strength of a temporary prosthesis and the material of a final prosthesis. Using this, the user may later adjust the A/H of the abutment corresponding to the third similarity in the second abutment library and select an optimized abutment using the modified value.

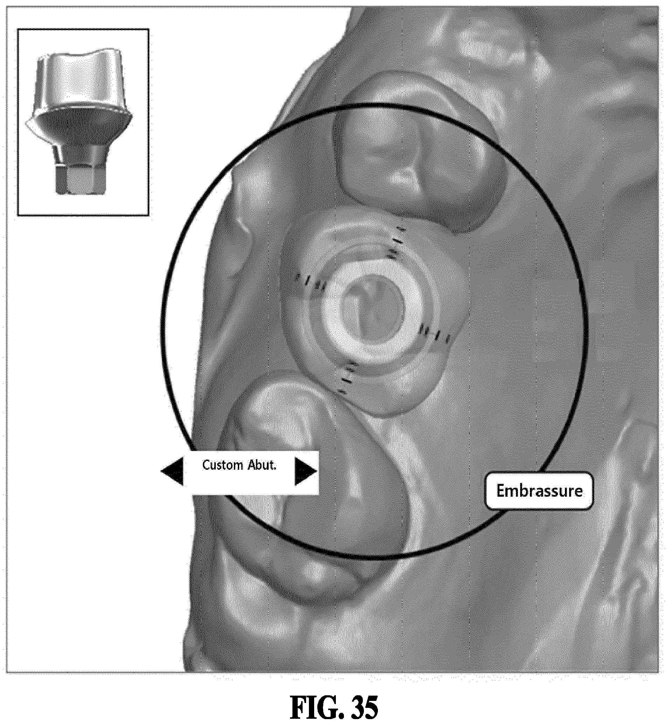

FIG. 35 is a screen presenting a specification of a patient-specific abutment and providing a user with an abutment selection screen through a virtual manipulator according to an embodiment of the present disclosure, and FIG. 36 is a screen presenting a specification of a matched ready-made abutment and providing a user with an abutment selection screen through a virtual manipulator according to an embodiment of the present disclosure.

Referring to FIG. 35, the user may check a designed patient-specific abutment through a screen and select it through a virtual manipulator.

Referring to FIG. 36, the software may provide the user with a plurality of ready-made abutments selected from the second abutment library along with product code and/or abutment specifications (e.g., mesiodistal (M/D) length, buccolingual (B/L) length, etc.), and the user may then select one from among the ready-made abutments provided through a virtual manipulator. In this case, the software may display differently a product screen corresponding to a most suitable abutment to the user among the ready-made abutments selected from the second abutment library by highlighting the product screen or displaying an edge color thereof differently.

Hereinafter, a process of designing an abutment performed regardless of whether it is a process of designing a customized abutment will be described.

Referring back to FIG. 2, in step S700, when the user adjusts the placement position of the virtual implant body through the virtual manipulator, the software may check whether the placement position of the virtual implant body is changed. When a change in the placement position of the implant body is checked ("Yes" in S700), the software may place the virtual crown again in step S800 and check whether it is a process of designing a patient-specific abutment in step S400. Based on a result of the checking, the software may perform the process of designing a ready-made abutment or the process of designing a patient-specific abutment and recommend an abutment to the user in steps S500 and S600. Subsequently, the user may select an abutment whose specifications have been changed in real time using the virtual manipulator.

Accordingly, the user may receive a recommended optimal abutment according to a movement of the implant body in real time without using separate software.

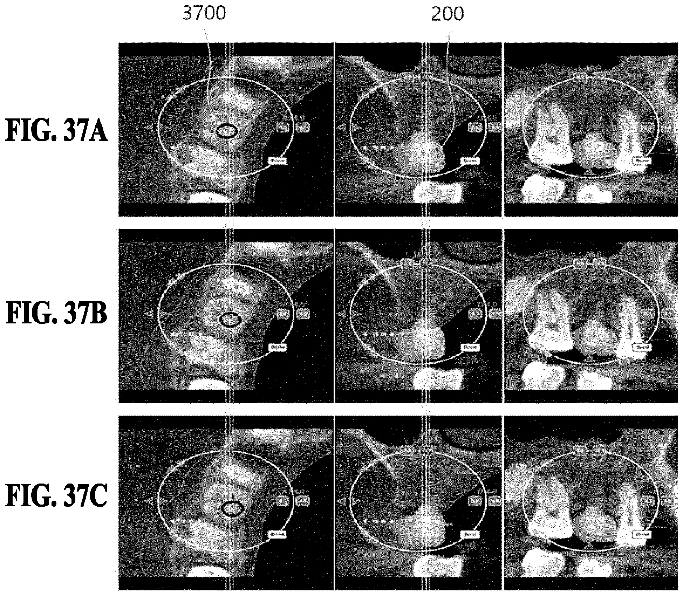

FIG. 37 is a screen providing a user with a change in the shape of a virtual crown in response to a change in a placement position of an implant body according to an embodiment of the present disclosure.

Referring to FIGS. 2 and 37, in step S700, the software may check a change in a placement position of a virtual implant body and, when the placement position (e.g., 3700) of the virtual implant body is moved, may change the shape of a virtual crown 200 according to the movement of the placement position 3700 of the virtual implant body and provide the changed shape to the user.

Therefore, the user may immediately check in real time the shape of the virtual crown changed according to a change in the placement position of the virtual implant body.

Each block of the block diagrams and each step of the flowcharts, and combinations thereof, of the accompanying drawings of the present disclosure may be implemented as computer programs or codes. The computer programs or codes may generate a means of performing the functions described in each block of the block diagrams or each step of the flowcharts. Since the computer programs or codes may be stored in any type of computer-usable or computer-readable recording medium, it may be possible to produce manufactured items that perform the functions described in each block of the block diagrams or each step of the flowcharts. In addition, the computer programs or codes may be provided in a computer or other programmable data processing equipment, such that they may perform a series of the described steps.

Each block or each step may represent a module or portion of code that includes one or more executable programs or codes, and the blocks and steps described herein may be performed in different orders that are not described herein. For example, two blocks or steps shown in succession may be performed simultaneously, or the blocks or steps may be performed in reverse order depending on the corresponding functions.

The description is provided merely to illustrate the technical idea of the present disclosure, and various modifications and changes may be made without departing from the essential quality of the present disclosure. Accordingly, the embodiments described herein are not intended to limit the technical idea of the present disclosure but to explain it, and the scope of the technical idea of the present disclosure is not limited by these embodiments. The scope of protection of the present disclosure shall be interpreted in accordance with the claims below, and all technical ideas within the scope equivalent thereto shall be construed as being included in the scope of the claims of the present disclosure.

## Claims

1. A method of recommending a virtual abutment for designing a surgical guide, the method comprising:
by a design device,
placing a virtual crown in dental image data;
designing a virtual implant body according to the placement of the virtual crown;
when the virtual abutment is set as a patient-specific abutment, determining at least one of an outer length of a gingival portion of the patient-specific abutment, an outer shape of the gingival portion, a diameter, or an abutment height A/H, based on the virtual crown and the virtual implant body; and
searching a prestored abutment library for an abutment matching the patient-specific abutment based on the determining.

2. The method of claim 1, wherein the searching comprises:
calculating a similarity between the patient-specific abutment and each abutment comprised in the abutment library, based on the determining; and
determining the abutment matching the patient-specific abutment from the abutment library, based on the similarity.

3. The method of claim 2, wherein the calculating of the similarity comprises:
calculating at least one of a first similarity between a gingival height G/H of the patient-specific abutment and a G/H of each abutment comprised in the abutment library, a second similarity between a diameter value of the patient-specific abutment and a diameter value of each abutment comprised in the abutment library, or a third similarity between the A/H of the patient-specific abutment and an A/H of each abutment comprised in the abutment library.

4. The method of claim 3, wherein the determining of the abutment matching the patient-specific abutment comprises:
comparing the calculated at least one of the first similarity, the second similarity, or the third similarity to a preset reference value for the at least one, and determining the abutment matching the patient-specific abutment.

5. The method of claim 4, further comprising:
when an A/H of the abutment determined to match the patient-specific abutment is greater than the A/H of the patient-specific abutment, modifying the A/H of the abutment determined to match the patient-specific abutment, based on at least one of a strength of a temporary prosthesis or a material of a final prosthesis.

6. The method of claim 1, further comprising:
when an abutment comprised in an implant is set as a ready-made abutment, searching the prestored abutment library for an abutment matching the ready-made abutment, based on an angle between a vertical line from an uppermost end of the virtual implant body to the virtual crown and a line from the uppermost end of the virtual implant body to a margin portion of the virtual crown.

7. The method of claim 1 or 6, further comprising:
when a position of the virtual implant body is moved, adjusting at least one of the outer length of the gingival portion of the patient-specific abutment, the outer shape of the gingival portion, the diameter, or the A/H, based on the moved position of the virtual implant body.

8. The method of claim 7, further comprising:
when the position of the virtual implant body is moved, changing a shape of the virtual crown based on the moved position of the virtual implant body.

9. A method of generating a surgical guide performed by a design device, the method comprising:
placing a virtual crown in dental image data;
designing a virtual implant body according to the placement of the virtual crown;
when a virtual abutment is set as a patient-specific abutment, determining at least one of an outer length of a gingival portion of the patient-specific abutment, an outer shape of the gingival portion, a diameter, or an abutment height A/H, based on the virtual crown and the virtual implant body;
searching a prestored abutment library for an abutment matching the patient-specific abutment based on the determining; and
applying the retrieved abutment to the surgical guide.

10. A method of recommending a virtual abutment for designing a surgical guide, the method comprising:
by a design device,
receiving an input as to whether the virtual abutment is a patient-specific abutment;
placing a virtual crown in dental image data;
designing a virtual implant body according to the placement of the virtual crown;
verifying whether the inputted virtual abutment is set as the patient-specific abutment; and
based on the placed virtual crown and the placed virtual implant body, searching a prestored abutment library for an abutment matching the patient-specific abutment when the virtual abutment is verified as the patient-specific abutment, and searching a prestored abutment library for an abutment matching a ready-made abutment when the virtual abutment is not verified as the patient-specific abutment.

11. A recommendation device, comprising:
a data acquisition unit configured to obtain dental image data; and
a control unit configured to: when a virtual abutment is set as a patient-specific abutment, place a virtual crown using the image data; design a virtual implant body according to the placement of the virtual crown; determine at least one of an outer length of a gingival portion of the patient-specific abutment, an outer shape of the gingival portion, a diameter, or an abutment height A/H, based on the virtual crown and the virtual implant body; and search a prestored abutment library for an abutment matching the patient-specific abutment based on the determining.

12. A non-transitory computer-readable recording medium storing a computer program,
wherein the computer program comprises:
instructions causing a processor to perform the method of any one of claims 1 to 10.

13. A computer program embodied on a non-transitory computer readable medium, the computer program being configured to control a processor to perform the method of any one of claims 1 to 10.
